(19) Europäisches Patentamt
European Patent Office
Office européen des brevets

(11) **EP 4 722 723 A1**

(12) **EUROPEAN PATENT APPLICATION**
published in accordance with Art. 153(4) EPC

(43) Date of publication:
**08.04.2026   Bulletin 2026/15**

(21) Application number: **24815384.3**

(22) Date of filing: **24.05.2024**

(51) International Patent Classification (IPC):
***G01N 33/545*** (2006.01)   ***G01N 33/543*** (2006.01)

(52) Cooperative Patent Classification (CPC):
**G01N 33/543; G01N 33/545**

(86) International application number:
**PCT/JP2024/019126**

(87) International publication number:
**WO 2024/247900 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **31.05.2023   JP 2023089651**

(71) Applicant: **NIPPON STEEL CHEMICAL &
MATERIAL CO., LTD.
Tokyo 103-0027 (JP)**

(72) Inventors:
• **MATSUMURA, Yasufumi
Tokyo 103-0027 (JP)**
• **ENOMOTO, Yasushi
Tokyo 103-0027 (JP)**
• **SHINTA, Ryuzo
Tokyo 103-0027 (JP)**

(74) Representative: **Becker, Eberhard
Becker Kurig & Partner
Patentanwälte mbB
Bavariastraße 7
80336 München (DE)**

(54) **LABELING SUBSTANCE, IMMUNOASSAY METHOD, IMMUNOASSAY REAGENT, METHOD FOR MEASURING ANALYTE, ANALYTE MEASUREMENT KIT, AND LATERAL-FLOW CHROMATOGRAPHIC TEST STRIP**

(57)    This labeling substance comprises a metal-resin composite 100 that has a resin particle 10 and a plurality of metal particles 20 immobilized on the resin particle 10. When the CV value of the particle diameter D1 of the metal-resin composite 100 is 6% or less and the mode for the particle diameter of the metal-resin composite is $\alpha$ [nm], the abundance rate of particles having a particle diameter less than $\alpha \times 0.94$ is 10% or less. In an immunoassay, the amount of analyte contained in a sample is quantified on the basis of the localized surface plasmon resonance of the metal-resin composite 100 in the labeling substance and/or a coloring intensity derived from light energy absorption caused by electron transitions.

FIG. 1

EP 4 722 723 A1

**Description**

Technical Field

The present invention relates to, for example, a labeling substance used in immunoassay, an immunoassay method, an immunoassay reagent, a method for measuring an analyte, an analyte measurement kit, and a lateral-flow chromatographic test strip.

Background Art

Immunoassay is a method that utilizes specific reaction between antigen and antibody to qualitatively and quantitatively analyze trace components. In an immunoassay, detection sensitivity is increased by binding a labeling substance to an antibody, an antigen, or composites thereof. Therefore, the labeling capability of the labeling substance can be said to be an important factor that influences the detection capability in an immunoassay. It has been proposed to use composite particles that combine metal particles and resin particle as a labeling substance to be used in an immunoassay (Patent Documents 1 to 3).

Patent Document 1 proposes colored latex composed of gold nanoparticles bound to the surfaces of polymer-based latex particles. Patent Documents 2 and 3 propose metal-resin composites in which a specific proportion of metal particles exist in the surface layer portion of a resin particle, which excel in durability and visibility, and enable highly sensitive determination in immunoassays without the need to add special equipment or work processes.

There are also proposals that enable quantification of analytes by immunoassays (Patent Documents 4 to 6). Patent Document 4 discloses a reverse lateral flow immunoassay method designed to detect human antigen-specific immunoglobulin E (IgE). Patent Document 5 discloses a method for determining the amount of analyte in a sample based on the signal obtained by irradiating a light beam to a lateral flow strip that uses highly doped upconversion nanoparticles. Patent Document 6 discloses a method for quantifying an analyte on a lateral flow assay (LFA) by applying a sample to a device that includes an LFA with a porous matrix and an aqueous two-phase system (ATPS) arranged in the porous matrix. In Patent Documents 4 to 6, studies have been conducted to improve the detection sensitivity of analytes by using ingenious instruments and reagents or by using special nanoparticles. However, it cannot be said that a sufficient correlation between the concentration of the analyte in the sample and the color development intensity has been obtained, and it is considered insufficient for performing quantification of the analyte with high accuracy.

Non-Patent Document 1 discloses a microgel in which gold nanoparticles are supported on poly-2-vinylpyridine latex particles, and confirms the pH responsiveness of the particle diameter of the microgel from changes in the behavior of localized surface plasmon resonance of the gold nanoparticles. However, in the above microgel, the gold nanoparticles are supported in a single layer near the surface layer of the latex particles. Therefore, the loading amount of gold nanoparticles is small, and it is considered that a deep color tone effective for immunoassays cannot be obtained. Besides, no studies have been conducted on the material, structure, composition, etc. of the microgel, and the effects on specific applications such as immunoassay reagents are unclear.

Related Art Documents

Patent Documents

Patent Document 1: Japanese Patent Application Laid-Open No. 2009-168495
Patent Document 2: International Publication WO2016/002742
Patent Document 3: International Publication WO2017/010391
Patent Document 4: Japanese Patent Publication No. 2023-506679
Patent Document 5: Japanese Patent Publication No. 2022-502637
Patent Document 6: Japanese Patent No. 6833519

Non-Patent Documents

Non-Patent Document 1: K. Akamatsu, M. Shimada, T. Tsuruoka, H. Nawafune, S. Fujii and Y. Nakamura; Langmuir 2010, 26, 1254-1259.

SUMMARY OF INVENTION

Problem to be Solved by the Invention

The present invention aims to provide a labeling substance with which it is possible to obtain a sufficient correlation between the concentration of an analyte in a sample and the color development intensity in an immunoassay, and to quantify the analyte with high accuracy. Means for Solving the Problem

As a result of intensive research, the present inventors found that the above problems can be solved by controlling the coefficient of variation (CV value) of the particle diameter of a metal-resin composite with a specific structure used as a labeling substance in an immunoassay, and completed the present invention.

That is, the labeling substance of the present invention is a labeling substance including a metal-resin composite that includes a resin particle and multiple metal particles immobilized on the resin particle. And, in the labeling substance of the present invention, a CV value of a particle diameter of the metal-resin composite is 6% or less, and in a case where a mode value of the particle diameter of the metal-resin composite detected by a particle size distribution analyzer is $\alpha$ [nm], an abundance ratio of particles having a particle diameter smaller than $\alpha \times 0.94$ is 10% or less.

In the labeling substance of the present invention, the metal particles may be particles of platinum or an alloy thereof, or particles of gold or an alloy thereof.

In the labeling substance of the present invention, an average particle diameter of the metal-resin composite may be within a range of 50 nm to 1000 nm.

In the labeling substance of the present invention, at least some of the metal particles may be distributed three-dimensionally in a surface layer portion of the resin particle.

The labeling substance of the present invention may be used by adsorbing an antigen or antibody on a surface of the metal-resin composite.

An immunoassay method of the present invention uses the above labeling substance.

An immunoassay reagent of the present invention includes the above labeling substance.

A method for measuring an analyte of the present invention is a method for detecting or quantifying the analyte contained in a sample. The method for measuring an analyte of the present invention uses a lateral-flow chromatographic test strip that includes a membrane and a determination section in which a capture ligand that specifically binds to the analyte is immobilized on the membrane. The method for measuring an analyte of the present invention includes the following steps (I) to (III);

　　　step (I): a step of bringing the analyte contained in the sample into contact with a labeled antibody in which an antibody that specifically binds to the analyte is labeled with the labeling substance,
　　　step (II): a step of bringing a composite including the analyte and the labeled antibody, formed in step (I), into contact with the capture ligand in the determination section, and
　　　step (III): a step of measuring a color development intensity derived from light energy absorption due to localized surface plasmon resonance and/or electron transition of the metal-resin composite in the labeling substance.

The method for measuring an analyte of the present invention may further include step (IV): a step of estimating an amount of analyte contained in the sample based on the measured color development intensity.

An analyte measurement kit of the present invention is provided for detecting or quantifying an analyte contained in a sample, and includes: a lateral-flow chromatographic test strip including a membrane and a determination section in which a capture ligand that specifically binds to the analyte is immobilized on the membrane; and a detection reagent including a labeled antibody in which an antibody that specifically binds to the analyte is labeled with the labeling substance.

The analyte measurement kit of the present invention may further include a device that measures a color development intensity in the determination section, and the color development intensity may be derived from light energy absorption due to localized surface plasmon resonance and/or electron transition of the metal-resin composite in a composite of the analyte and the labeled antibody.

The analyte measurement kit of the present invention may further include reference standard data showing a relationship between the color development intensity in the determination section and a concentration of the analyte, and the color development intensity may be derived from light energy absorption due to localized surface plasmon resonance and/or electron transition of the metal-resin composite in a composite of the analyte and the labeled antibody.

A lateral-flow chromatographic test strip of the present invention is provided for detecting or quantifying an analyte contained in a sample, and includes: a membrane; a determination section in which a capture ligand that specifically binds to the analyte is immobilized on the membrane in a direction in which the sample develops; and a reaction section including a labeled antibody in which an antibody that specifically binds to the analyte is labeled with the labeling substance on an upstream side of the determination section.

Effects of the Invention

Using the labeling substance of the present invention makes it possible to obtain a sufficient correlation between the concentration of the analyte and the color development intensity in an immunoassay, and to detect the analyte in the sample with high sensitivity, and in particular, highly accurate quantification becomes possible even for a low-concentration analyte.

Furthermore, since the labeling substance of the present invention has a structure in which multiple metal particles are immobilized on a resin particle, the loading amount of the metal particles is large, and the metal particles are less likely to detach from the resin particle. Moreover, the metal particles exhibit light energy absorption due to electron transition in addition to localized surface plasmon resonance.

Therefore, the labeling substance of the present invention excels in durability and visibility, and can be preferably applied to various immunoassays as an excellent material that enables highly sensitive detection of an analyte, particularly highly accurate quantification even for a low-concentration analyte, without the need to add special equipment or work processes.

BRIEF DESCRIPTION OF THE DRAWINGS

[FIG. 1] is a schematic diagram showing a cross-sectional structure of a metal-resin composite used in a labeling substance according to one embodiment of the present invention.
[FIG. 2A] is a schematic diagram showing a cross-sectional structure of one aspect of the metal-resin composite.
[FIG. 2B] is a schematic diagram showing a cross-sectional structure of another aspect of the metal-resin composite.
[FIG. 3] is an explanatory diagram showing an overview of a method for measuring an analyte using a lateral-flow chromatographic test strip according to one embodiment of the present invention.
[FIG. 4] is a cross-sectional view of an immunochromatographic strip prepared in the examples.
[FIG. 5A] is a scatter plot of test line color development intensity versus CRP antigen concentration in Examples 1 to 3 and Comparative Examples 1 to 3.
[FIG. 5B] is a scatter plot of test line color development intensity versus CRP antigen concentration in an antigen concentration range of 0 ng/ml to 5 ng/ml in Examples 1 to 3 and Comparative Examples 1 to 3.

DESCRIPTION OF THE EMBODIMENTS

Hereinafter, embodiments of the present invention will be described in detail with appropriate reference to the drawings.

[Labeling Substance]

The labeling substance of the present embodiment includes a metal-resin composite, and is applied to an immunoassay. The metal-resin composite has a resin particle and multiple metal particles immobilized on the resin particle. The CV value of the particle diameter of the metal-resin composite is 6% or less. By setting the CV value to 6% or less, as shown in the examples described later, a strong correlation is obtained between the concentration of an analyte in a sample and the color development intensity in immunochromatography. As a result, it is possible to detect an analyte with high sensitivity, and particularly highly accurate quantification becomes possible even for a low-concentration analyte. From such a viewpoint, the CV value of the particle diameter of the metal-resin composite is preferably 5% or less, more preferably 4% or less, even more preferably 3% or less, and most preferably 2% or less.

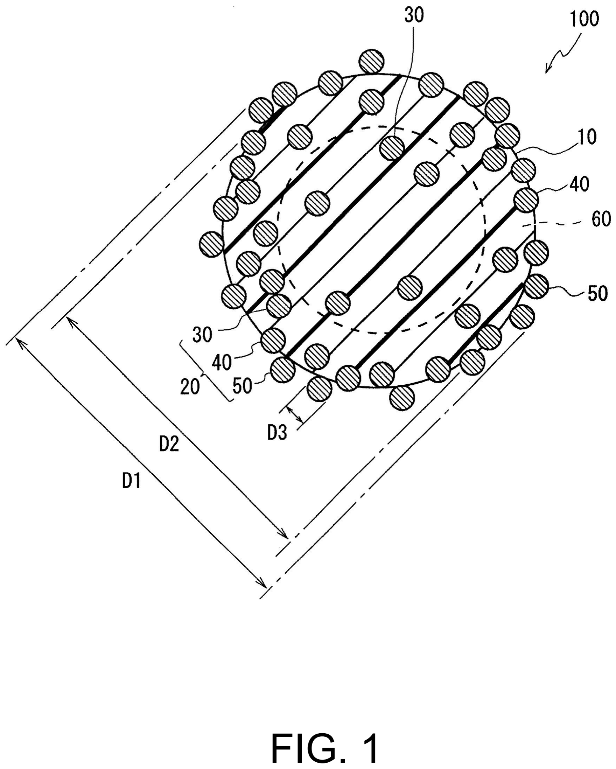

FIG. 1 is an example of a cross-sectional schematic diagram of a metal-resin composite. A metal-resin composite 100 includes a resin particle 10 and metal particles 20. In the metal-resin composite 100, the metal particles 20 are immobilized on the resin particle 10. The resin particle 10 is preferably a relatively larger particle than the metal particles 20. In other words, in the metal-resin composite 100, a large number of relatively small metal particles 20 are immobilized on the large resin particle 10. In this case, as shown in FIG. 1, the relationship among a particle diameter D1 of the metal-resin composite 100, a particle diameter D2 of the resin particle 10, and a particle diameter D3 of the metal particles 20 is D1>D2>D3. Here, the particle diameter D1 of the metal-resin composite 100 means the maximum diameter based on the metal particles 20 positioned at the outermost side in the metal-resin composite 100. That is, the particle diameter D1 of the metal-resin composite 100 is a value obtained by adding the length of the protruding portion of partially exposed particles 40 or surface adsorbed particles 50 described later to the particle diameter D2 of the resin particle 10.

The average particle diameter of the metal-resin composite 100 (that is, the average of the particle diameter D1 in FIG. 1) is preferably, for example, 50 nm to 1000 nm. In the case of the average particle diameter of the metal-resin composite 100 being smaller than 50 nm, for example, the loading amount of the metal particles 20 tends to decrease, so coloration tends to be weaker than coloration of metal particles 20 of the same size; and in the case of exceeding 1000 nm, the metal-resin composite 100 used as a labeling substance or reagent has a tendency to easily clog in the pores of the chromatographic medium such as a membrane filter and a tendency to decrease dispersibility. The upper limit of the average particle diameter of the metal-resin composite 100 is preferably 600 nm, and more preferably 500 nm, from the viewpoint of improving dispersibility for use as a labeling substance or reagent, and obtaining high detection sensitivity in the case of using the metal-resin composite 100 for immunoassay. On the other hand, the lower limit is preferably 100 nm, more preferably 200 nm, and even more preferably 300 nm. In particular, in the case of the average particle diameter of the metal-resin composite 100 being 300 nm or more, using the metal-resin composite 100 as a labeling substance for immunochromatography achieves stable and excellent detection sensitivity. The average particle diameter of the metal-resin composite 100 can be measured by a laser diffraction/scattering method, dynamic light scattering method, or centrifugal sedimentation method.

Further, the metal-resin composite 100 has an abundance ratio of 10% or less for particles having a particle diameter smaller than $\alpha \times 0.94$ in the case where the mode value of the particle diameter (that is, D1 in FIG. 1) detected by a particle size distribution analyzer is $\alpha$ [nm]. This abundance ratio is preferably 5% or less, more preferably 3% or less, and even more preferably 2% or less.

By setting the abundance ratio of the metal-resin composite 100 having a particle diameter smaller than $\alpha \times 0.94$ to 10% or less, the light absorption performance per particle of the metal-resin composite 100 excellent in color development can be aligned to substantially the same level. As a result, high sensitivity and quantitative color development can be obtained with respect to antigen concentration in immunochromatography, which is preferable.

For example, a centrifugal sedimentation particle size distribution analyzer can be adopted as the particle size distribution analyzer.

In addition, the mode value $\alpha$ of particle diameter is the particle diameter with the highest detection frequency in the particle size distribution of the metal-resin composite 100 measured by the particle size distribution analyzer. Further, $\alpha \times 0.94$ is a particle diameter obtained by multiplying $\alpha$ by 0.94 in the case of the mode value of the particle diameter of the metal-resin composite 100 being $\alpha$ [nm]. Here, the reason for multiplying by 0.94 is that in the case of the particle diameter being too small compared to the mode value, the loading amount of metal becomes less compared to the metal-resin composite having a particle diameter at the mode value, which results in a small color development intensity. This is because an excessively high ratio of such particles with a small color development intensity causes the detection sensitivity to decrease. That is, in order to have high detection sensitivity, it is preferable to have fewer particles with a particle diameter smaller than the mode value of particle diameter.

<Resin Particle>

The resin particle 10 is not limited in structure or composition, but is preferably a particle of a polymer having substituents in the structure that are capable of adsorbing metal ions. Examples of the polymer having substituents in the structure that are capable of adsorbing metal ions include polymers capable of adsorbing anionic ions and polymers capable of adsorbing cationic ions.

As polymers capable of adsorbing anionic ions, nitrogen-containing polymers are particularly preferable. Nitrogen-containing polymers are resins having nitrogen atoms in the main chain or side chain, and examples include polyamine, polyamide, polypeptide, polyurethane, polyurea, polyimide, polyimidazole, polyoxazole, polypyrrole, polyaniline, etc. Polyamines such as poly-2-vinylpyridine, poly-3-vinylpyridine, and poly-4-vinylpyridine are preferable. Further, in the case of having nitrogen atoms in the side chain, for example, acrylic resins, phenol resins, epoxy resins, etc. can be widely used. The nitrogen atoms in the nitrogen-containing polymer easily chemisorb anionic ions such as $[PtCl_6]^{2-}$ and $[AuCl_4]^-$, which are precursors of the metal particles 20 that excel in visibility and facilitate immobilization of antigens or antibodies. In the present embodiment, the metal ions adsorbed in the nitrogen-containing polymer are reduced to form the metal particles 20, so some of the formed metal particles 20 become encapsulated particles 30 or partially exposed particles 40. In the case of using $[PtCl_6]^{2-}$, platinum particles are formed, and in the case of using $[AuCl_4]^-$, gold particles are formed.

Further, polymers capable of adsorbing cationic ions are resins having carboxylic acid groups, sulfonic acid groups, etc. in the main chain or side chain. Specific examples of polymers capable of adsorbing cationic ions include carboxylic acid group-containing polymers such as acrylic acid polymers and sulfonic acid group-containing polymers such as polystyrene sulfonic acid. More specifically, polyacrylic acid, vinyl carboxylic acid, polyvinyl acetate, polyvinyl sulfonic acid, polystyrene sulfonic acid, etc. can be mentioned. Polymers capable of adsorbing cationic ions can chemisorb cationic ions such as $Pt^{2+}$ and $Au^{3+}$ by the carboxylic acid groups, sulfonic acid groups, etc. contained, and therefore are preferable. For example, by reducing the chemisorbed $Pt^{2+}$ and $Au^{3+}$ to form the metal particles 20 (in this case, platinum particles or gold particles), it is possible to produce a structure similar to the above nitrogen-containing polymer particles.

Polymers other than the polymer having substituents capable of adsorbing metal ions in the structure include, for example, polystyrene, etc., but in this case, it is difficult to adsorb metal ions inside the resin particle 10. As a result, most of the generated metal particles 20 become surface adsorbed particles 50. As described later, since the surface adsorbed particles 50 have a small contact area with the resin particle 10, the adhesive force between the resin particle 10 and the metal particles 20 is small, and the metal particles 20 tend to easily detach from the resin particle 10.

The polymer having substituents capable of adsorbing metal ions in the structure may be copolymers with known polymerizable monomers. Here, examples of the copolymers include random copolymers, block copolymers, alternating copolymers, and copolymers in which polymers are crosslinked with each other. In addition, two or more types of monomers may be copolymerized to form the resin particle 10, or monomers may be reacted with functional groups present on the surface of the resin particle 10 and further polymerized using these as polymerization active terminals. The copolymerization composition is not limited, but it is preferable that the monomer containing substituents capable of adsorbing the metal ions is 10 mol% or more. The polymerizable monomers can be selected without restriction according to the application of the metal-resin composite 100. For example, in applications for improving the shape, size uniformity, and dispersion stability of the resin particle 10, polymerizable monomers having characteristics as surfactants can be used. Examples of such polymerizable monomers include polyethylene glycol methyl ether methacrylate and polyethylene glycol dimethacrylate.

Further, the average particle diameter of the resin particle 10 (that is, the average of the particle diameter D2 in FIG. 1) is preferably, for example, 45 nm to 900 nm. In the case of the average particle diameter of the resin particle 10 being less than 45 nm, the variation in the particle diameter D1 of the metal-resin composite 100 becomes large, making it difficult to control the CV value to 6% or less. On the other hand, in the case of the average particle diameter of the resin particle 10 exceeding 900 nm, the metal-resin composite 100 used as a labeling substance or reagent has a tendency to easily clog in the pores of the chromatographic medium such as a membrane filter and a tendency to decrease dispersibility.

From the viewpoint of obtaining high detection sensitivity in an immunoassay using a labeling substance and particularly enabling highly accurate quantification, the upper limit of the average particle diameter of the resin particle 10 is preferably 630 nm, more preferably 540 nm, and even more preferably 450 nm. On the other hand, the lower limit of the resin particle 10 is preferably 90 nm, more preferably 180 nm, and even more preferably 270 nm.

Additionally, in order to control the CV value of the particle diameter D1 of the metal-resin composite 100 to 6% or less, it is preferable to set the CV value of the particle diameter of the resin particle 10 to 5% or less. In the case of the CV value of the particle diameter of the resin particle 10 exceeding 5%, variation in the particle diameter D1 of the metal-resin composite 100 tends to occur, making it difficult to control the CV value to 6% or less. Therefore, the CV value of the particle diameter of the resin particle 10 is preferably 5% or less, more preferably 4% or less, even more preferably 3% or less, and most preferably 2% or less.

The average particle diameter of the resin particle 10 can be measured by a laser diffraction/scattering method, dynamic light scattering method, or centrifugal sedimentation method.

<Metal Particles>

The metal particles 20 in the metal-resin composite 100 are preferably particles of platinum or an alloy thereof (hereinafter may simply be referred to as "platinum particles") or particles of gold or an alloy thereof (hereinafter may simply be referred to as "gold particles").

A platinum-resin composite using platinum particles as the metal particles 20 is less likely to cause aggregation in a state of being bound with ligands such as antibodies compared to metal-resin composites having particles of other metal species, and has extremely excellent dispersibility.

Further, platinum particles are resistant to deterioration such as oxidation and have excellent storage stability. Furthermore, platinum particles exhibit absorption derived from localized surface plasmon resonance in a wide wavelength range of, for example, 250 nm to 900 nm, and further exhibit strong color development close to black due to the manifestation of light energy absorption by electron transition. Therefore, using the platinum-resin composite as a labeling substance can obtain high visibility in immunoassays, and also increase the detection sensitivity of analytes. By using platinum particles, excellent detection sensitivity can be obtained with a smaller loading amount compared to particles of other metals. Thus, assuming that the average particle diameters are equivalent, the platinum-resin composite shows significantly higher detection sensitivity compared to resin composites having particles of other metal species.

The platinum particles may be composed of platinum alone or may be an alloy of platinum and other metals. The platinum alloy means an alloy composed of platinum and metal species other than platinum, and containing platinum in an amount of 1% by weight or more, preferably 50% by weight or more, and more preferably 60% by weight or more. Here, other metal species that form an alloy with platinum are not particularly limited, but for example, gold, palladium, silver, nickel, copper, etc. are preferable, and gold, palladium, etc., which are excellent in storage stability and visibility, are more preferable.

A gold-resin composite using gold particles as metal particles is less likely to cause aggregation in a state of being bound with ligands such as antibodies compared to metal-resin composites having particles of other metal species, and has extremely excellent dispersibility. Furthermore, the gold-resin composite has excellent visibility and facilitates immobilization of antigens or antibodies. In particular, by controlling the particle diameter of gold particles and the inter-particle distance between gold particles, various color developments such as red, purple, and blue can be exhibited. Therefore, in the case of using the gold-resin composite as a labeling substance for immunochromatography, labeling substances of various colors can be obtained.

The gold particles may be composed of gold alone or may be an alloy of gold and other metals. The gold alloy means an alloy composed of gold and metal species other than gold, and containing gold in an amount of 1% by weight or more, preferably 50% by weight or more, and more preferably 60% by weight or more. Here, other metal species that form an alloy with gold are not particularly limited, but for example, platinum, palladium, silver, nickel, copper, etc. are preferable, and platinum, palladium, etc., which are excellent in storage stability and visibility, are more preferable.

Further, the metal particles 20 may have particles of other metals together with platinum particles or gold particles. As such metals, palladium, silver, nickel, and copper are preferable from the viewpoint of ease of nano-size composite formation of metals and resins. These metals can be used as simple substances or alloys.

In addition, the average particle diameter of the metal particles 20 measured by scanning electron microscope (SEM) observation (that is, the average of the particle diameter D3 in FIG. 1) is preferably, for example, 1 nm to 80 nm in the case of platinum particles. In the case of the average particle diameter of platinum particles being less than 1 nm or exceeding 80 nm, localized surface plasmon resonance and light energy absorption by electron transition are less likely to be exhibited, so sensitivity tends to decrease.

Further, the upper limit of the average particle diameter of platinum particles is preferably 50 nm, more preferably 30 nm, even more preferably 20 nm, and most preferably 15 nm, from the viewpoint of obtaining high detection sensitivity as a labeling substance. In particular, in the case of the average particle diameter of platinum particles being 15 nm or less, particularly excellent detection sensitivity can be obtained in using the metal-resin composite 100 as a labeling substance for immunochromatography. On the other hand, the lower limit of platinum particles is preferably 2 nm.

In addition, the average particle diameter of the metal particles 20 measured by scanning electron microscope (SEM) observation is preferably, for example, 1 nm to 80 nm in the case of gold particles. In the case of the average particle diameter of gold particles being less than 1 nm or exceeding 80 nm, localized surface plasmon resonance and light energy absorption by electron transition are less likely to be exhibited, so sensitivity tends to decrease.

Further, the upper limit of the average particle diameter of gold particles is preferably 70 nm, and more preferably 50 nm, from the viewpoint of obtaining high detection sensitivity as a labeling substance. On the other hand, the lower limit of gold particles is preferably 2 nm.

In order to control the CV value of the particle diameter D1 of the metal-resin composite 100 to 6% or less, it is preferable to set the standard deviation of the particle diameter of the metal particles 20 nm to 15 nm or less. In the case of the standard deviation of the particle diameter of the metal particles 20 exceeding 15 nm, variation in the particle diameter D1 of the metal-resin composite 100 is likely to occur, and it becomes difficult to control the CV value to 6% or less. Therefore, the standard deviation of the particle diameter of the metal particles 20 is preferably 5 nm or less, more preferably 3 nm or less, and even more preferably 2 nm or less, in the case of platinum particles. Further, in the case of gold particles, the standard deviation of the particle diameter is preferably 14 nm or less, more preferably 13 nm or less, and even more preferably 12 nm or less.

<Existence State of Metal Particles>

The existence state of the metal particles 20 in the metal-resin composite 100 is not limited. For example, the metal particles 20 may be distributed two-dimensionally on the surface of the resin particle 10, or the metal particles 20 may be encapsulated inside the resin particle 10. Further, a core-shell structure may be formed with the resin particle 10 as a shell and the metal particles 20 as a core.

In addition, some of the metal particles 20 may be distributed three-dimensionally inside the resin particle 10 or in a surface layer portion 60. In this case, some of the three-dimensionally distributed metal particles 20 may be partially exposed outside the resin particle 10, and some of the remaining metal particles 20 may be encapsulated in the resin particle 10. Specifically, as shown in FIG. 1, it is preferable that the metal particles 20 include metal particles completely encapsulated in the resin particle 10 (also referred to as "encapsulated particles 30"), and metal particles having a portion embedded in the resin particle 10 and a portion exposed outside the resin particle 10 (also referred to as "partially exposed particles 40"). In addition to this, metal particles adsorbed on the surface of the resin particle 10 (also referred to as "surface adsorbed particles 50") may be present.

Here, "surface layer portion" means a range of 50% of the particle radius in the depth direction from the surface of the resin particle 10, based on the outermost position of the metal-resin composite 100 (that is, the protruding end portion of the partially exposed particles 40 or the surface adsorbed particles 50). Further, "three-dimensionally distributed" means that the metal particles 20 are dispersed not only in the surface direction of the resin particle 10 but also in the depth direction.

In the case of using the metal-resin composite 100 as a labeling substance, an antigen, antibody, or blocking agent is immobilized on the surface of the resin particle 10 or on the surface of the partially exposed particles 40 or the surface adsorbed particles 50 for use. At this time, while the antigen, antibody, or blocking agent is immobilized on the partially exposed particles 40 and the surface adsorbed particles 50, it is considered difficult for the antigen, antibody, or blocking agent to be immobilized on the encapsulated particles 30. However, since the partially exposed particles 40, the surface adsorbed particles 50, and the encapsulated particles 30 all exhibit light energy absorption by electron transition in addition to localized surface plasmon resonance, not only the partially exposed particles 40 and the surface adsorbed particles 50 but also the encapsulated particles 30 contribute to improving the visibility of the labeling substance. Furthermore, the partially exposed particles 40 and the encapsulated particles 30 have a larger contact area with the resin particle 10 compared to the surface adsorbed particles 50, and the embedded state also achieves an anchor effect, so the physical adsorption force is strong and makes it difficult for the partially exposed particles 40 and the encapsulated particles 30 to detach from the resin particle 10. In addition, the antigen, antibody, or blocking agent adsorbed on the partially exposed particles 40 or the surface adsorbed particles 50 forms coordination bonds with the metal, making it difficult to detach. Therefore, the labeling substance using the metal-resin composite 100 can achieve excellent durability and stability.

In the metal-resin composite 100, the encapsulated particles 30 have the entire surface covered with the resin that constitutes the resin particle 10. Further, the partially exposed particles 40 have 5% or more and less than 100% of the surface area covered with the resin that constitutes the resin particle 10, and from the viewpoint of durability of the labeling substance, the lower limit is preferably 20% or more of the surface area, and more preferably 30% or more. Further, the surface adsorbed particles 50 preferably have more than 0% and less than 5% of the surface area covered with the resin that constitutes the resin particle 10.

Moreover, the loading amount of the metal particles 20 (total of the encapsulated particles 30, the partially exposed particles 40, and the surface adsorbed particles 50) in the metal-resin composite 100 is preferably 5% by weight to 70% by weight relative to the weight of the metal-resin composite 100. Within this range, the metal-resin composite 100 excels in visibility, visual determination, and detection sensitivity as a labeling substance. In the case of the loading amount of the metal particles 20 being less than 5% by weight, the immobilization amount of antibody or antigen becomes small, and the detection sensitivity tends to decrease. The lower limit of the loading amount of the metal particles 20 is more preferably 10% by weight, and even more preferably 15% by weight, and the upper limit is more preferably 70% by weight, and even more preferably 65% by weight. The metal-resin composite 100 having platinum particles or gold particles as the metal particles 20 can obtain excellent visibility, visual determination, and detection sensitivity as a labeling substance even with a smaller loading amount compared to other metal particles.

Further, it is preferable that 10% by weight to 90% by weight of the metal particles 20 are the partially exposed particles 40 and the surface adsorbed particles 50. Within this range, a sufficient immobilization amount of antibody or antigen can be secured on the metal particles 20, so the sensitivity as a labeling substance is high. It is more preferable that 20% by weight to 80% by weight of the metal particles 20 are the partially exposed particles 40 and the surface adsorbed particles 50, and even more preferable that the surface adsorbed particles 50 are 20% by weight or less from the viewpoint of durability of the labeling substance.

Further, in the case of using the metal-resin composite 100 for immunoassay, in order to obtain excellent detection sensitivity, it is preferable that 60% by weight to 100% by weight, preferably 75% by weight to 100% by weight, and more preferably 85% by weight to 100% by weight of the metal particles 20 are present in the surface layer portion 60, and it is even more preferable that the metal particles 20 are present within a range of 40% of the particle radius in the depth direction from the surface of the resin particle 10. Further, it is preferable that 5% by weight to 90% by weight of the metal particles 20 present in the surface layer portion 60 are the partially exposed particles 40 or the surface adsorbed particles 50 for a sufficient immobilization amount of antibody or antigen can be secured on the metal particles 20, resulting in high sensitivity as a labeling substance. In other words, it is preferable that 10% by weight to 95% by weight of the metal particles 20 present in the surface layer portion 60 are the encapsulated particles 30.

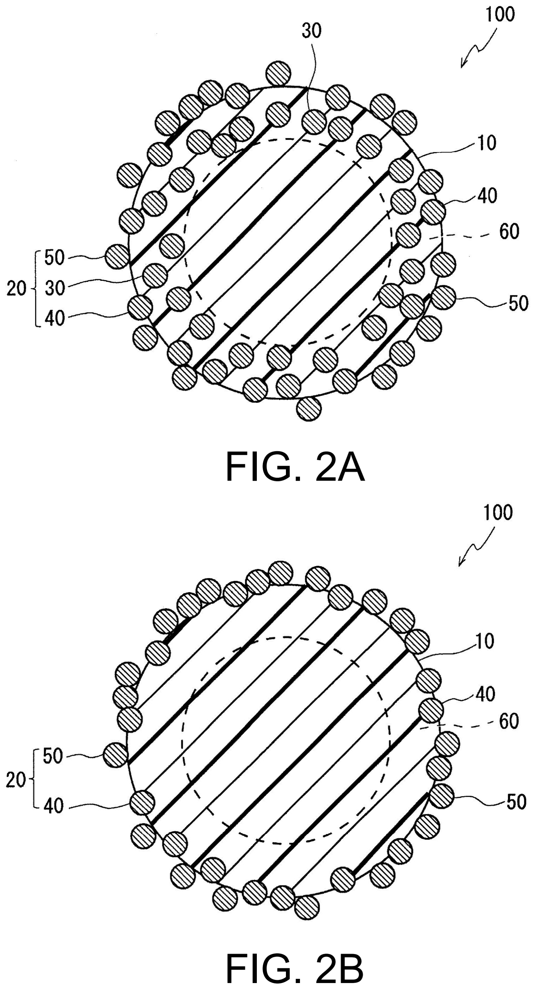

As described above, since the encapsulated particles 30 also exhibit light energy absorption by electron transition in addition to localized surface plasmon resonance, not only the partially exposed particles 40 and the surface adsorbed particles 50, but also the encapsulated particles 30 contribute to improving the visibility of the labeling substance. From the viewpoint of such visibility improvement, it is preferable that the encapsulated particles 30 are concentrated and distributed in the metal-resin composite 100 within a certain range in the depth direction from the surface of the resin particle 10, as shown in FIG. 2A, for example, and no encapsulated particles 30 are present near the center of the resin particle 10. More specifically, in order to effectively exhibit light energy absorption by electron transition in addition to localized surface plasmon resonance by the encapsulated particles 30, for example, in the case of the particle diameter D2 of the resin particle 10 being 800 nm, it is preferable that 70% by weight or more, preferably 80% by weight or more, more preferably 90% by weight to 100% by weight of the encapsulated particles 30 are present within a range of, for example, 0 nm to 200 nm in the depth direction from the surface of the resin particle 10. In particular, in the case of the region where all (100% by weight) of the encapsulated particles 30 are distributed (encapsulated particle distribution region) being within a range of, for example, 0 nm to 100 nm from the surface of the resin particle 10, it is preferable because light energy absorption by electron transition can be maximized in addition to localized surface plasmon resonance by the encapsulated particles 30.

In addition, the metal-resin composite 100 may not have the encapsulated particles 30. For example, as shown in FIG. 2B, in the metal-resin composite 100, all of the metal particles 20 may be immobilized on the surface of the resin particle 10 without overlapping in the radial direction of the resin particle 10. In this case, the metal particles 20 are composed of the partially exposed particles 40 and/or the surface adsorbed particles 50.

[Method for Manufacturing Metal-Resin Composite]

The method for manufacturing the metal-resin composite 100 is not particularly limited. For example, a solution containing metal ions is added to a dispersion liquid of the resin particles 10 manufactured by an emulsion polymerization method to adsorb the metal ions to the resin particles 10 (hereinafter referred to as "metal ion-adsorbed resin particles"). Furthermore, by adding the metal ion-adsorbed resin particles to a reducing agent solution, the metal ions can be reduced to generate metal particles 20, thereby obtaining the metal-resin composite 100.

In the case of generating platinum particles as the metal particles 20, examples of the solution containing platinum ions include a chloroplatinic acid ($H_2PtCl_6$) aqueous solution, platinum chloride ($PtCl_2$) solution, etc. Further, a platinum complex may be used instead of platinum ions.

Additionally, in the case of generating gold particles as the metal particles 20, examples of the solution containing gold ions include a chloroauric acid ($HAuCl_4$) aqueous solution, etc. Further, a gold complex may be used instead of gold ions.

Further, as the solvent for the solution containing metal ions, hydrous alcohols or alcohols such as methanol, ethanol, n-propanol, isopropanol, n-butanol, sec-butanol, and t-butanol, or acids such as hydrochloric acid, sulfuric acid, and nitric acid may be used instead of water.

Furthermore, additives such as water-soluble polymer compounds like polyvinyl alcohol, surfactants, alcohols; ethers such as tetrahydrofuran, diethyl ether, and diisopropyl ether; polyols such as alkylene glycol, polyalkylene glycol, monoalkyl ethers or dialkyl ethers thereof, and glycerin; and various water-miscible organic solvents such as ketones like acetone, and methyl ethyl ketone may be added to the solution as required. Such additives are effective for promoting the reduction reaction rate of metal ions and controlling the size of the generated metal particles 20.

Further, known reducing agents can be used. Examples include sodium borohydride, dimethylamine borane, citric acid, sodium hypophosphite, hydrazine hydrate, hydrazine hydrochloride, hydrazine sulfate, formaldehyde, sucrose, glucose, ascorbic acid, erythorbic acid, sodium phosphinate, hydroquinone, Rochelle salt, etc. Among these, sodium borohydride, dimethylamine borane, or citric acid is preferable.

A surfactant can be added to the reducing agent solution as required, or the pH of the solution can be adjusted. The pH adjustment can be performed using buffer agents such as boric acid and phosphoric acid, acids such as hydrochloric acid and sulfuric acid, or alkalis such as sodium hydroxide and potassium hydroxide.

Furthermore, the particle diameter of the metal particles 20 to be formed can be controlled by adjusting the reduction rate of metal ions according to the temperature of the reducing agent solution.

Further, in reducing the metal ions in the metal ion-adsorbed resin particles to generate the metal particles 20, the metal ion-adsorbed resin particles may be added to the reducing agent solution, or the reducing agent may be added to the metal ion-adsorbed resin particles.

Further, in order to maintain the dispersibility of the metal-resin composite 100 in water, dispersants such as citric acid, poly-L-lysine, polyvinylpyrrolidone, polyvinylpyridine, polyvinyl alcohol, DISPERBYK194, DISPERBYK180, and DISPERBYK184 (manufactured by BYK Chemie Japan) may be added.

Furthermore, the pH can be adjusted using buffer agents such as boric acid and phosphoric acid, acids such as hydrochloric acid and sulfuric acid, or alkalis such as sodium hydroxide and potassium hydroxide to maintain dispersibility.

Depending on the manufacturing conditions of the metal-resin composite 100, variation in the particle diameter D1 of the metal-resin composite 100 may occur, resulting in a large CV value exceeding 6%. In that case, it is difficult to perform high-sensitivity detection, particularly highly accurate quantification.

In order to control the CV value of the particle diameter D1 of the metal-resin composite 100 to 6% or less, it is preferable to first uniformize the particle diameter D2 of the resin particle 10. For this purpose, it is important to sufficiently mix and homogenize the reaction solution containing raw material monomers, surfactants, polymerization catalysts, etc. in emulsion polymerization of the resin particle 10. For example, it is preferable to sufficiently stir the reaction solution containing raw material monomers, surfactants, polymerization catalysts, etc. before adding the polymerization initiator. The stirring speed depends on the reaction scale, but for example, in the case of using a 500-milliliter round container and stirring with a plate-shaped stirring blade having an incomplete semicircular shape (a shape obtained by cutting a circle at a position other than the center) [lateral width (length of cut edge) 8 cm, vertical width 1.6 cm, thickness 0.3 cm; hereinafter simply referred to as "incomplete semicircular stirring blade"] and mounted so that the longitudinal direction is perpendicular to the central axis of the round container, 30 rpm to 500 rpm, preferably 50 rpm to 300 rpm, and more preferably 100 rpm to 200 rpm is suitable, and the stirring speed is preferably constant. In the case of the stirring speed being less than 30 rpm, the dispersion state of the raw material monomers becomes non-uniform, and in the case of exceeding 500 rpm, the flow of the reaction solution becomes turbulent, and in either case, the particle diameter D2 of the resin particle 10 becomes non-uniform. At this time, since the stirring temperature and stirring time are also important for aligning the particle diameter D2 of the resin particle 10, it is preferable to perform stirring while stepwise changing the temperature and time between, for example, room temperature to about 70°C. More specifically, in the process of heating the reaction solution, it is preferable to perform sufficient stirring for about 20 minutes to 60 minutes each in temperature ranges of, for example, around 25°C to 35°C and around 55°C to 65°C.

The combination of the reaction vessel and stirrer can be appropriately selected to achieve stirring efficiency equivalent to the above.

Further, after adding the polymerization initiator to the reaction solution, it is preferable to sufficiently stir the reaction solution in a temperature range of, for example, around 55°C to 65°C. The stirring speed depends on the reaction scale, but for example, in the case of using a 500-milliliter round container and stirring with an incomplete semicircular stirring blade, 30 rpm to 500 rpm, preferably 50 rpm to 300 rpm, and more preferably 100 rpm to 200 rpm is suitable. Then, in the case of the stirring speed being less than 30 rpm, the dispersion state of the raw material monomers becomes non-uniform, and in the case of exceeding 500 rpm, the flow of the reaction solution becomes turbulent, and in either case, the particle diameter D2 of the resin particle 10 becomes non-uniform. The stirring time is, for example, about 1 hour to 5 hours, and preferably about 2 hours to 4 hours.

The combination of the reaction vessel and stirrer can be appropriately selected to achieve stirring efficiency equivalent to the above.

Furthermore, in order to control the CV value of the particle diameter D1 of the metal-resin composite 100 to 6% or less, it is also important to sufficiently mix and homogenize the resin particle dispersion liquid including the resin particle 10 and the metal compound. The resin particle dispersion liquid depends on the reaction scale, but for example, in the case of using a 500-milliliter round container and stirring with an incomplete semicircular stirring blade, it is preferable to sufficiently stir at a speed of 30 rpm to 500 rpm, preferably 50 rpm to 300 rpm, and more preferably 100 rpm to 200 rpm, and the stirring speed is preferably constant. In addition, since the stirring temperature and stirring time of the resin particle dispersion liquid are also important for aligning the particle diameter D1 of the metal-resin composite 100, it is preferable to perform stirring in a temperature range of, for example, room temperature to about 40°C, and preferably about 25°C to 35°C, for a time of, for example, about 1 hour to 5 hours, and preferably about 2 hours to 4 hours. By sufficiently homogenizing the resin particle dispersion liquid before adding the reducing agent, the metal ions become homogeneously adsorbed to the resin particle 10, making it possible to reduce the standard deviation of the particle diameter of the metal particles 20, and as a result, it becomes easy to control the CV value of the particle diameter D1 of the metal-resin composite 100 to 6% or less.

The combination of the reaction vessel and stirrer can be appropriately selected to achieve stirring efficiency equivalent to the above.

Additionally, in order to control the CV value of the particle diameter D1 of the metal-resin composite 100 to 6% or less, it is preferable to sufficiently stir the mixed solution (reduction reaction solution) of the resin particle dispersion liquid and the reducing agent in the reduction step. The stirring speed depends on the reaction scale, but for example, in the case of using a 500-milliliter round container and stirring with an incomplete semicircular stirring blade, it is preferable to stir at a speed of 30 rpm to 500 rpm, preferably 50 rpm to 300 rpm, and more preferably 100 rpm to 200 rpm, and the stirring speed is preferably constant. At this time, the stirring temperature and stirring time are also important for aligning the particle diameter D1 of the metal-resin composite 100. For example, in the process of heating the reaction solution from a state cooled to around 0°C to near room temperature, it is preferable to perform sufficient stirring for about 30 minutes to 120 minutes in a temperature range of about 0°C to 5°C and for about 1 hour to 3 hours in a temperature range of about 25°C to 30°C. By sufficiently homogenizing the reduction reaction solution, it becomes possible to reduce the standard deviation of the particle diameter of the metal particles 20, and as a result, it becomes easy to control the CV value of the particle diameter D1 of the metal-resin composite 100 to 6% or less.

The combination of the reaction vessel and stirrer can be appropriately selected to achieve stirring efficiency equivalent to the above.

The metal-resin composite 100 obtained as described above can be preferably applied to various immunoassays as a labeling substance, particularly by adsorbing an antigen or antibody on the surface of the metal particles 20. In addition, since the CV value of the particle diameter D1 of the metal-resin composite 100 is controlled to 6% or less, the metal-resin composite 100 not only excels in visual determination in low concentration ranges (high sensitivity regions), but can also be preferably applied as a labeling substance capable of quantitative analysis even for low-concentration analytes by using a measuring device such as an immunochromatography reader, or as an immunoassay reagent including such a labeling substance. Further, although there is no particular limitation on the forms of the labeling substance and the immunoassay reagent, for example, the labeling substance and the immunoassay reagent can be used as a dispersion liquid in which the metal-resin composite 100 is dispersed in water or a buffer with adjusted pH.

The method for adsorbing an antigen or antibody on the surface of the metal particles 20 is not particularly limited, and known methods based on physical adsorption and chemisorption can be used. Since the bond between the metal particles 20 and the antigen or antibody is strong, a method based on chemisorption is preferable. Physical adsorption and chemisorption may also be used in combination.

Examples of physical adsorption include a method of immersing the metal-resin composite 100 in a buffer including an antigen or antibody and incubating, a method of immersing the metal-resin composite 100 in a buffer and further adding an antigen or antibody, etc.

Examples of chemisorption include a method of introducing an SH group into an antigen or antibody and reacting the SH group with the metal-resin composite 100 to form a metal-SH bond, a method of introducing a carboxyl group on the surface of the metal-resin composite 100, then succinimidylating the carboxyl group and reacting the carboxyl group with an amino group of an antigen or antibody to form a chemical bond, etc. Compounds for introducing a carboxyl group on the surface of the metal-resin composite 100 are suitably compounds having both a functional group with coordination bonding properties to metals, such as amino groups, SH groups, carbonyl groups, amide groups, and imide groups, and a carboxyl group. Since the coordination bonds with metals are strong, SH groups are preferable as the functional group, and examples of compounds having an SH group and a carboxyl groups at both terminals include mercaptopropionic acid, mercaptoundecanoic acid, mercaptolauric acid, etc. In addition, polymer compounds having multiple functional groups with coordination bonding properties to metals and carboxyl groups within one molecule can easily obtain stable bonds with the metal-resin composite 100. For example, polyglutamic acid and polyaspartic acid, which are polypeptides of glutamic acid and aspartic acid, are more preferable as polymer compounds for introducing a carboxyl group on the surface of the metal-resin composite 100.

Next, the method for measuring an analyte using the metal-resin composite 100 as a labeling substance, a lateral-flow chromatographic test strip, and an analyte detection/quantification kit will be described.

[Lateral-Flow Chromatographic Test Strip]

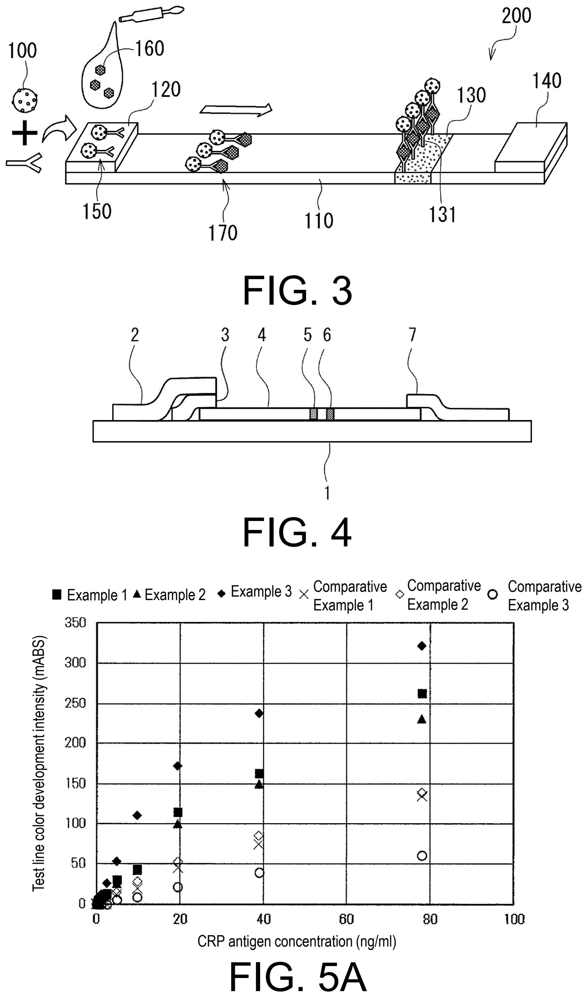

First, a lateral-flow chromatographic test strip (hereinafter, may be simply referred to as "test strip") according to one embodiment of the present invention will be described with reference to FIG. 3. This test strip 200 can be preferably used in the method for measuring an analyte according to one embodiment of the present invention, as described later.

The test strip 200 includes a membrane 110. The membrane 110 is provided with a sample addition section 120, a determination section 130, and a liquid absorption section 140 in this order in the sample development direction.

<Membrane>

Membranes used as the membrane materials in general test strips can be applied as the membrane 110 used in the test strip 200. The membrane 110 is formed of an inert substance (a substance that does not react with an analyte 160, various ligands, etc.) composed of a fine porous material that exhibits, for example, capillary action and allows the sample to develop simultaneously with the addition of the sample. Specific examples of the membrane 110 include fibrous or non-woven fibrous matrices, membranes, filter papers, glass fiber filter papers, cloths, cotton, etc. composed of polyurethane, polyester, polyethylene, polyvinyl chloride, polyvinylidene fluoride, nylon, cellulose derivatives, and the like. Among these, membranes, filter papers, glass fiber filter papers, etc. composed of cellulose derivatives or nylon are preferably used, and nitrocellulose membranes, mixed nitrocellulose ester (mixture of nitrocellulose and cellulose acetate) membranes, nylon membranes, and filter papers are more preferably used.

The test strip 200 preferably includes a support that supports the membrane 110 in order to make the operation more convenient. For example, plastic or the like can be used as the support.

<Sample Addition Section>

The test strip 200 may have the sample addition section 120 for adding a sample including the analyte 160. The sample addition section 120 is a site for receiving the sample including the analyte 160 in the test strip 200. The sample addition section 120 may be formed on the membrane 110 on the upstream side of the determination section 130 in the direction in which the sample develops, or a sample addition pad composed of a material such as cellulose filter paper, glass fiber, polyurethane, polyacetate, cellulose acetate, nylon, and cotton cloth may be provided on the membrane 110 to constitute the sample addition section 120.

<Determination Section>

In the determination section 130, a capture ligand 131 that specifically binds to the analyte 160 is immobilized. The capture ligand 131 can be used without particular limitation as long as the capture ligand 131 forms a specific bond with the analyte 160, and for example, an antibody against the analyte 160 can be preferably used. The capture ligand 131 is immobilized so that the capture ligand 131 does not move from the determination section 130 even in the case of providing a sample to the test strip 200. The capture ligand 131 may be directly or indirectly immobilized to the membrane 110 by physical or chemical bonding, adsorption, etc.

Further, the determination section 130 is not particularly limited as long as the determination section 130 has a configuration that brings a composite 170 including a labeled antibody 150 and the analyte 160 into contact with the capture ligand 131 that specifically binds to the analyte 160. For example, the capture ligand 131 may be directly immobilized to the membrane 110, or the capture ligand 131 may be immobilized to a pad composed of cellulose filter paper, glass fiber, non-woven fabric, etc. that is immobilized to the membrane 110.

<Liquid Absorption Section>

The liquid absorption section 140 is formed by, for example, a pad of a water-absorbing material such as cellulose filter paper, non-woven fabric, cloth, and cellulose acetate. The movement speed of the sample after the development front line of the added sample reaches the liquid absorption section 140 varies depending on the material, size, and other factors of the liquid absorption section 140. Therefore, by selecting the material, size, and other factors of the liquid absorption section 140, an optimal speed for detection and quantification of the analyte 160 can be set. The liquid absorption section 140 is an optional configuration and may be omitted.

The test strip 200 may further include optional sites such as a reaction section and a control section as required.

<Reaction Section>

Although not illustrated, the test strip 200 may have a reaction section that includes the labeled antibody 150 formed on the membrane 110. The reaction section can be provided on the upstream side of the determination section 130 in the direction in which the sample flows. The sample addition section 120 in FIG. 3 may be used as the reaction section. In the case of the test strip 200 having the reaction section, in response to a sample including the analyte 160 being supplied to the reaction section or the sample addition section 120, the analyte 160 contained in the sample and the labeled antibody 150 can be brought into contact with each other in the reaction section. In this case, the composite 170 including the analyte 160 and the labeled antibody 150 can be formed by simply supplying the sample to the reaction section or the sample addition section 120, so that so-called one-step immunochromatography becomes possible.

The reaction section is not particularly limited as long as the reaction section includes the labeled antibody 150 that specifically binds to the analyte 160, but the reaction section may also be formed by directly applying the labeled antibody 150 to the membrane 110. Alternatively, the reaction section may be formed by immobilizing to the membrane 110 a pad (conjugate pad) composed of, for example, cellulose filter paper, glass fiber, non-woven fabric, etc. impregnated with the labeled antibody 150.

<Control Section>

Although not illustrated, the test strip 200 may have a control section formed on the membrane 110 on the downstream side of the determination section 130 in the direction in which the sample develops, where a capture ligand that specifically binds to the labeled antibody 150 is immobilized. By measuring the color development intensity at both the determination section 130 and the control section, it can be confirmed that the sample supplied to the test strip 200 has developed and reached the reaction section and the determination section 130, and that the test has been performed normally. The control section is prepared in the same manner as the above-described determination section 130, except that another type of capture ligand that specifically binds to the labeled antibody 150 is used instead of the capture ligand 131, and can adopt the same configuration.

[Method for Measuring Analyte]

Next, a method for measuring an analyte 160 according to one embodiment of the present invention performed using a test strip 200 will be described.

The method for measuring the analyte 160 according to the present embodiment is a measurement method of the analyte 160 for detecting or quantifying the analyte 160 contained in a sample. The method for measuring the analyte 160 according to the present embodiment uses the test strip 200 that includes a membrane 110 and a determination section 130 in which a capture ligand 131 that specifically binds to the analyte 160 is immobilized on the membrane 110. The method for measuring the analyte 160 according to the present embodiment can include the following steps (I) to (III);

step (I): a step of bringing the analyte 160 contained in the sample into contact with a labeled antibody 150 in which an antibody that specifically binds to the analyte 160 is labeled with a labeling substance including a metal-resin composite 100,
step (II): a step of bringing a composite 170 including the analyte 160 and the labeled antibody 150 formed in step (I) into contact with the capture ligand 131 in the determination section 130, and
step (III): a step of measuring a color development intensity derived from light energy absorption due to localized surface plasmon resonance and/or electron transition of the metal-resin composite 100 in the labeling substance.

Furthermore, the method for measuring the analyte 160 according to the present embodiment can include, as an optional step,
step (IV): a step of estimating the amount of analyte contained in the sample based on the measured color development intensity.

Step (I):

Step (I) is a step of bringing the analyte 160 contained in the sample into contact with the labeled antibody 150. The manner of contact is not particularly limited as long as the composite 170 including the analyte 160 and the labeled antibody 150 is formed. For example, the sample may be supplied to the sample addition section 120 or reaction section (not shown) of the test strip 200, and the analyte 160 may be brought into contact with the labeled antibody 150 in the sample addition section 120 or reaction section, or the analyte 160 in the sample may be brought into contact with the labeled antibody 150 before supplying the sample to the test strip 200.
The composite 170 formed in step (I) develops and moves on the test strip 200, and reaches the determination section 130.

Step (II):

Step (II) brings the composite 170 including the analyte 160 and the labeled antibody 150, which is formed in step (I), into contact with the capture ligand 131 in the determination section 130 of the test strip 200. In response to the composite 170 being brought into contact with the capture ligand 131, the capture ligand 131 specifically binds to the analyte 160 of the composite 170. As a result, the composite 170 is captured in the determination section 130.
Since the capture ligand 131 does not specifically bind to the labeled antibody 150, in the case of the labeled antibody 150 unbound to the analyte 160 reaching the determination section 130, the labeled antibody 150 unbound to the analyte 160 passes through the determination section 130. Here, in the case of the test strip 200 having a control section (not shown) in which another capture ligand that specifically binds to the labeled antibody 150 is immobilized, the labeled antibody 150 passing through the determination section 130 continues to develop and binds with the another capture ligand in the control section. As a result, the labeled antibody 150 that has not formed the composite 170 with the analyte 160 is captured in the control section.
After step (II), a washing step may be performed, before step (III) as required, to wash the test strip 200 with a buffer commonly used in biochemical tests, such as water, physiological saline, and phosphate buffer. The washing step can remove the labeled antibody 150 that has not been captured in the determination section 130, or in the determination section 130 and the control section (the labeled antibody 150 that is not bound to the analyte 160 and does not form the composite 170).
By performing the washing step, in step (III), the background color development intensity can be reduced in the case of measuring the color development due to light energy absorption caused by localized surface plasmon resonance and/or electron transition of the metal-resin composite 100 in the determination section 130, or in the determination section 130 and the control section, thereby increasing the signal/background ratio to further improve detection sensitivity and quantitativeness.

Step (III):

Step (III) is a step of measuring the color development intensity derived from light energy absorption due to localized surface plasmon resonance and/or electron transition of the metal-resin composite 100. After performing the above step (II) or the washing step as required, the analyte 160 contained in the sample can be detected with high sensitivity by measuring the color development intensity derived from light energy absorption due to localized surface plasmon resonance and/or electron transition of the metal-resin composite 100 in the determination section 130 of the test strip 200.

The measurement of color development intensity can be performed using a measuring device such as an immunochromatography reader. The measurement principle of color development intensity in the measuring device is not particularly limited. For example, the immunochromatography reader irradiates light to the determination section 130 and measures the color development intensity from the attenuation of reflected light.

In the case of a control section being formed on the test strip 200, by step (II), the labeled antibody 150 is captured by another capture ligand in the control section to form a composite. Therefore, in step (III), color development due to light energy absorption caused by localized surface plasmon resonance and/or electron transition can be generated not only in the determination section 130 but also in the control section of the test strip 200. By measuring the color development intensity in the determination section 130 and the control section in this manner, it can be confirmed whether the sample supplied to the test strip 200 has normally developed and reached the reaction section and the determination section 130.

Step (IV) is a quantification step of estimating the amount of analyte 160 contained in the sample based on the measured color development intensity.

Reference standard data showing the relationship between the color development intensity in the determination section 130 and the amount of analyte 160 can be used in this step. Examples of the reference standard data include a calibration curve, table, formula, etc. that shows the relationship between color development intensity and analyte concentration. The reference standard data may be data prepared for general use, or may be created by conducting preliminary experiments based on standard reagents with known analyte concentrations before the immunoassay. The labeling substance of the present embodiment has a high correlation between color development intensity and analyte concentration because the CV value of the particle diameter D1 of the metal-resin composite 100 is 6% or less. Specifically, in the calibration curve showing the relationship between color development intensity and analyte concentration, a strong correlation is shown even in the low concentration range. For example, the correlation coefficient becomes 0.90 or more, and preferably 0.95 or more. Therefore, it is possible to perform not only high-sensitivity detection for the analyte 160 but also highly accurate quantification.

<Sample and Analyte>

The sample in the method for measuring the analyte according to the present embodiment is not particularly limited as long as the sample includes a substance that can serve as an antigen, such as a protein, as the analyte 160. For example, biological samples including the target analyte 160 (that is, whole blood, serum, plasma, urine, saliva, sputum, nasal or pharyngeal swab fluid, cerebrospinal fluid, amniotic fluid, nipple discharge, tears, sweat, exudate from skin, extracts from tissues, cells, and feces, etc.), food extracts, etc., can be mentioned. In response to necessity, prior to the above step (I), the analyte 160 contained in the sample may be pretreated in order to facilitate the specific binding reaction between the labeled antibody 150 and capture ligand 131 and the analyte 160. Here, examples of pretreatment include chemical treatment using various chemicals such as acids, bases, and surfactants, and physical treatment using heating, stirring, and ultrasonic waves. Particularly, in the case of the analyte 160 being a substance that is not normally exposed on the surface, such as influenza virus NP antigen, it is preferable to perform treatment with a surfactant or the like. As the surfactant used for this purpose, a nonionic surfactant can be used in consideration of the binding reactivity between the capture ligand 131 and the analyte 160, such as specific binding reaction, for example, antigen-antibody reaction.

Further, the sample may be appropriately diluted with a solvent (water, physiological saline, or buffer, etc.) or a water-miscible organic solvent used in conventional immunoassay methods.

The analyte 160 is not particularly limited, and known analytes can be used, including analytes with strong anionic properties, analytes with strong cationic properties, and others. The analyte 160 is not particularly limited as long as the analyte 160 specifically binds to the labeled antibody 150 and the capture ligand 131, but examples include proteins (including polypeptides, oligopeptides, etc.) such as tumor markers, signal transduction substances, and hormones, nucleic acids (including single-stranded or double-stranded DNA, RNA, polynucleotides, oligonucleotides, PNA (peptide nucleic acid), etc.) or substances having nucleic acids, sugars (including oligosaccharides, polysaccharides, sugar chains, etc.) or substances having sugar chains, and other molecules such as lipids. More specifically, examples of the analyte include carcino-embryonic antigen (CEA), HER2 protein, prostate specific antigen (PSA), CA19-9, $\alpha$-fetoprotein (AFP), immunosuppressive acidic protein (IAP), CA15-3, CA125, estrogen receptor, progesterone receptor, fecal occult blood, troponin I, troponin T, CK-MB, CRP, human chorionic gonadotropin (HCG), luteinizing hormone (LH), follicle-stimulating hormone (FSH), syphilis antibody, influenza virus human hemoglobin, chlamydia antigen, group A $\beta$-hemolytic streptococcus antigen, HBs antibody, HBs antigen, rotavirus, adenovirus, albumin, glycated albumin, etc. Among these, antigens that are solubilized by nonionic surfactants are preferable, and antigens that form self-assemblies such as viral nucleoproteins are more preferable.

<Labeled Antibody>

The labeled antibody 150 is used in step (I) to contact with the analyte 160 contained in the sample to form the composite 170 including the analyte 160 and the labeled antibody 150. The labeled antibody 150 is formed by labeling an antibody that specifically binds to the analyte 160 with the metal-resin composite 100 having a structure in which multiple metal particles 20 are immobilized on the resin particle 10. Here, "labeling" means that in steps (I) to (III), the metal-resin composite 100 is immobilized on the antibody directly or indirectly by chemical or physical bonding, adsorption, etc. to such an extent that the metal-resin composite 100 does not detach from the labeled antibody 150. For example, the labeled antibody 150 may be formed by directly bonding the metal-resin composite 100 to the antibody, may be formed by bonding the antibody and the metal-resin composite 100 via an arbitrary linker molecule, or may be formed by the metal-resin composite 100 and the antibody being respectively immobilized to insoluble particles.

In addition, in the present embodiment, the "antibody" is not particularly limited and known antibodies can be used, including antibodies with strong anionic properties, antibodies with strong cationic properties, and others. For example, polyclonal antibodies, monoclonal antibodies, antibodies obtained by genetic recombination, and antibody fragments having binding ability to antigens [for example, H chain, L chain, Fab, $F(ab')_2$, etc.] can be used. As immunoglobulins, any of IgG, IgM, IgA, IgE, and IgD may be used. The animal species producing the antibody may be humans or non-human animals (for example, mice, rats, rabbits, goats, horses, etc.). Specific examples of the antibody include anti-PSA antibody, anti-AFP antibody, anti-CEA antibody, anti-adenovirus antibody, anti-influenza virus antibody, anti-HCV antibody, anti-IgG antibody, anti-human IgE antibody, etc.

<Preferred Method for Preparing Labeled Antibody>

Next, a preferred method for preparing the labeled antibody 150 will be described. The production of the labeled antibody 150 can include at least the following step A;

step A) a step of obtaining the labeled antibody 150 by mixing and binding the metal-resin composite 100 with an antibody under first pH conditions, and
preferably further include step B;
step B) a step of treating the labeled antibody 150 under second pH conditions.

[Step A]

In step A, the metal-resin composite 100 is mixed with an antibody under first pH conditions to obtain the labeled antibody 150. In step A, it is preferable to bring the solid metal-resin composite 100 into contact with the antibody in a state where the solid metal-resin composite 100 is dispersed in a liquid phase.

The first pH conditions are preferably conditions within a range of pH 2 to 10, and more preferably within a range of pH 5 to 9, for example, from the viewpoint of achieving uniform contact between the metal-resin composite 100 and the antibody while maintaining the dispersion of the metal-resin composite 100 and the activity of the antibody. In the case of conditions for binding the metal-resin composite 100 with the antibody being less than pH 2, the antibody may be altered and deactivated due to strong acidity, and in the case of exceeding pH 10, aggregation occurs during mixing of the metal-resin composite 100 and the antibody, making dispersion difficult. However, in the case of the antibody not being deactivated by strong acidity, treatment is possible even at less than pH 2.

Step A is preferably performed in a binding buffer adjusted to the first pH conditions. For example, a predetermined amount of the metal-resin composite 100 is mixed with the binding buffer adjusted to the above pH, and thoroughly mixed. For example, a boric acid solution adjusted to a predetermined concentration can be used as the binding buffer. The pH adjustment of the binding buffer can be performed using, for example, hydrochloric acid, sodium hydroxide, and the like.

Next, a predetermined amount of antibody is added to the obtained mixed solution, and sufficiently stirred and mixed to obtain the labeled antibody-containing solution. The labeled antibody-containing solution obtained in this manner can separate only the labeled antibody 150 as a solid portion by solid-liquid separation means such as centrifugation.

[Step B]

In step B, blocking is performed to suppress non-specific adsorption to the labeled antibody 150 by treating the labeled antibody 150 obtained in step A under second pH conditions. In this case, the labeled antibody 150 separated by the solid-liquid separation means is dispersed in a liquid phase under the second pH conditions.

The second pH conditions are preferably within a range of pH 2 to 10, for example, from the viewpoint of maintaining the activity of the antibody and suppressing aggregation of the labeled antibody 150, and more preferably within a range of pH 5 to 9 from the viewpoint of suppressing non-specific adsorption of the labeled antibody 150. In the case of blocking conditions being less than pH 2, the antibody may be altered and deactivated due to strong acidity, and in the case of exceeding pH 10, the labeled antibody 150 aggregates, making dispersion difficult.

Step B is preferably performed using a blocking buffer adjusting a blocking agent to the second pH conditions. For example, the blocking buffer adjusted to the above pH is added to a predetermined amount of the labeled antibody 150, and the labeled antibody 150 is uniformly dispersed in the blocking buffer. As the blocking buffer, for example, it is preferable to use a solution of protein that does not bind to the analyte. The blocking agent usable in the blocking buffer is not particularly limited and known blocking agents can be used, including blocking agents with strong anionic properties, blocking agents with strong cationic properties, and others. For example, in the case of proteins, bovine serum albumin, egg white albumin, casein, gelatin, whey, etc. can be mentioned. More specifically, it is preferable to use a bovine serum albumin solution adjusted to a predetermined concentration. The pH adjustment of the blocking buffer can be performed using, for example, hydrochloric acid, sodium hydroxide, and the like. For dispersion of the labeled antibody 150, it is preferable to use dispersion means such as ultrasonic treatment. In this way, a dispersion liquid in which the labeled antibody 150 is uniformly dispersed is obtained.

In the above step A and step B, the metal-resin composite 100 is less likely to aggregate due to pH and can be treated over a wide range of pH from acidic to alkaline. Therefore, the metal-resin composite 100 used in the present invention also has the advantage of being less subject to restrictions on the preparation conditions of the labeled antibody 150.

As described above, a dispersion liquid of the labeled antibody 150 is obtained. It is possible to separate only the labeled antibody 150 as a solid portion from this dispersion liquid by solid-liquid separation means such as centrifugation. Additionally, washing treatment, storage treatment, and the like can be performed as required. Hereinafter, the washing treatment and storage treatment will be described.

(Washing Treatment)

In the washing treatment, a washing buffer is added to the labeled antibody 150 separated by the solid-liquid separation means, and the labeled antibody 150 is uniformly dispersed in the washing buffer. For dispersion, it is preferable to use dispersion means such as ultrasonic treatment. The washing buffer is not particularly limited, but for example, Tris buffer, glycine amide buffer, arginine buffer, and the like adjusted to a predetermined concentration within a pH range of 8 to 9 can be used. The pH adjustment of the washing buffer can be performed using, for example, hydrochloric acid, sodium hydroxide, and the like. The washing treatment of the labeled antibody 150 can be repeated multiple times as required.

(Storage Treatment)

In the storage treatment, a storage buffer is added to the labeled antibody 150 separated by the solid-liquid separation means, and the labeled antibody 150 is uniformly dispersed in the storage buffer. For dispersion, it is preferable to use dispersion means such as ultrasonic treatment. As the storage buffer, for example, a solution obtained by adding a predetermined concentration of an anti-aggregation agent and/or a stabilizer to the washing buffer can be used. As the anti-aggregation agent, for example, saccharides represented by sucrose, maltose, lactose, and trehalose, and polyhydric alcohols represented by glycerin and polyvinyl alcohol can be used. The stabilizer is not particularly limited, but for example, proteins such as bovine serum albumin, egg white albumin, casein, and gelatin can be used. The storage treatment of the labeled antibody 150 can be performed in this manner.

In each of the above steps, surfactants and preservatives such as sodium azide and paraoxybenzoic acid esters can be further used as required.

[Analyte Measurement Kit]

An analyte measurement kit according to one embodiment of the present invention is a kit for detecting or quantifying the analyte 160 contained in a sample based on the method for measuring an analyte of the present embodiment using, for example, a test strip 200.

The analyte measurement kit of the present embodiment includes:

a test strip 200, including a membrane 110, and
a determination section 130 in which a capture ligand 131 that specifically binds to the analyte 160 is immobilized on the membrane 110; and
a detection reagent, including a labeled antibody 150 in which an antibody that specifically binds to the analyte 160 is labeled with a labeling substance including a metal-resin composite 100.

The analyte measurement kit of the present embodiment may further include other components as required. For example, the analyte measurement kit can include a measuring device that measures the color development intensity in the determination section 130. The analyte measurement kit can also include reference standard data such as a calibration curve, table, and formula that shows the relationship between color development intensity and analyte concentration.

In using the analyte measurement kit according to the present embodiment, after performing step (I) by bringing the analyte 160 in the sample into contact with the labeled antibody 150 in the detection reagent, the sample may be supplied to the reaction section or the sample addition section 120 of the test strip 200, and steps (II), (III), and (IV) may be performed sequentially. Alternatively, the detection reagent may be applied to the upstream side of the determination section 130 of the test strip 200 and appropriately dried to form a reaction section, and then the sample may be added to the formed reaction section or a position on the upstream side of the reaction section (for example, the sample addition section 120), and steps (I) to (IV) may be performed sequentially.

Examples

Next, the present invention will be specifically described by examples. Nevertheless, the present invention is not limited by these examples in any aspect. In the following examples and comparative examples, various measurements and evaluations are performed according to the following methods, unless otherwise specified.

<Absorbance Measurement of Platinum-Resin Composite Particles>

The absorbance of the platinum-resin composite particles was measured by placing a platinum-resin composite particle dispersion liquid (dispersion medium: water) prepared to 0.01 wt% (weight%) in a quartz glass cell (optical path length 10 mm) and measuring the absorbance at 400 nm using a spectrophotometer (UV3600, manufactured by Shimadzu Corporation).

<Absorbance Measurement of Gold-Resin Composite Particles>

The absorbance of the gold-resin composite particles was measured by placing a gold-resin composite particle dispersion liquid (dispersion medium: water) prepared to 0.01 wt% (weight%) in a quartz glass cell (optical path length 10 mm) and measuring the absorbance at 570 nm using a spectrophotometer (UV3600, manufactured by Shimadzu Corporation).

<Solid Content Concentration Measurement and Measurement of Metal Loading Amount>

1 g of the metal-resin composite particle dispersion liquid before concentration adjustment was placed in a porcelain crucible and dried at 70°C for 3 hours. The weight before and after drying was measured, and the solid content concentration was calculated using the following formula.

$$\text{Solid content concentration (wt\%)} =$$

$$[\text{Weight after drying (g)} / \text{Weight before drying (g)}] \times 100$$

Furthermore, the sample after the above drying treatment was further subjected to heat treatment at 500°C for 3 hours, the weight before and after heat treatment was measured, and the metal loading amount was calculated using the following formula.

$$\text{Metal loading amount (wt\%)} =$$

$$[\text{Weight after heat treatment (g)} / \text{Weight before heat treatment (g)}] \times 100$$

<Measurement of Average Particle Diameter of Metal Particles>

A substrate prepared by dropping the metal-resin composite particle dispersion liquid onto a metallic mesh with carbon support film was observed using a field emission scanning electron microscope (FE-SEM; manufactured by Hitachi High-Technologies Corporation, SU-9000), and the area average diameter of arbitrary 100 metal particles was measured from the obtained image to determine the average particle diameter.

<Measurement of Average Particle Diameter of Resin Particle and Metal-Resin Composite Particles, Calculation of CV Value and Mode Value>

The measurement was performed using a centrifugal sedimentation particle size distribution analyzer (LUMi-Sizer610 manufactured by LUM GmbH). The measurement was performed with particles dispersed in water or solution.
The coefficient of variation CV value was calculated from the average particle diameter and standard deviation obtained by measurement using the following formula.

$$\text{CV value (\%)} = \text{Standard deviation (nm) / Average particle diameter (nm)} \times 100$$

[Preparation Example 1]

<Synthesis of Resin Particles>

A 500-milliliter round container was used, and an incomplete semicircular stirring blade was mounted so that the longitudinal direction was perpendicular to the central axis of the round container. Into this round container, 300 g of pure water, 80 wt% trioctylmethylammonium chloride aqueous solution (1.55 g, 3.07 mmol), and 50 wt% polyethylene glycol methyl ether methacrylate aqueous solution (10.00 g, 2.40 mmol) were added and dissolved. Next, 2-vinylpyridine (48.00 g, 457 mmol) and divinylbenzene (2.00 g, 15.4 mmol) were added, and the mixture was stirred under nitrogen flow at 30°C for 50 minutes, then at 60°C for 30 minutes at a stirring speed of 100 rpm to 200 rpm. After stirring, 2,2-azobis(2-methylpropionamidine) dihydrochloride (0.250 g, 0.922 mmol) dissolved in 18.00 g of pure water was added dropwise, and the mixture was stirred at 60°C for 3 hours at a stirring speed of 100 rpm to 200 rpm to obtain resin particles A-1. The particles were precipitated by centrifugation, the supernatant was removed, and then the particles were redispersed in pure water, followed by removal of impurities through filtration treatment. Thereafter, concentration adjustment was performed to obtain a 10 wt% resin particle dispersion liquid B-1. The average particle diameter of resin particles A-1 in resin particle dispersion liquid B-1 was 395 nm, and the CV value was 1.44%.

[Preparation Example 2]

<Synthesis of Resin Particles>

A 500-milliliter round container was used, and an incomplete semicircular stirring blade was mounted so that the longitudinal direction was perpendicular to the central axis of the round container. Into this round container, 300 g of pure water, 80 wt% trioctylmethylammonium chloride aqueous solution (1.76 g, 3.48 mmol), and 50 wt% polyethylene glycol methyl ether methacrylate aqueous solution (10.00 g, 2.40 mmol) were added and dissolved. Next, 2-vinylpyridine (48.00 g, 457 mmol) and divinylbenzene (2.00 g, 15.4 mmol) were added, and the mixture was stirred under nitrogen flow at 30°C for 50 minutes, then at 60°C for 30 minutes at a stirring speed of 100 rpm to 200 rpm. After stirring, 2,2-azobis(2-methylpropionamidine) dihydrochloride (0.250 g, 0.922 mmol) dissolved in 18.00 g of pure water was added dropwise, and the mixture was stirred at 60°C for 3 hours at a stirring speed of 100 rpm to 200 rpm to obtain resin particles A-2. The particles were precipitated by centrifugation, the supernatant was removed, and then the particles were redispersed in pure water, followed by removal of impurities through filtration treatment. Thereafter, concentration adjustment was performed to obtain a 10 wt% resin particle dispersion liquid B-2. The average particle diameter of resin particles A-2 in resin particle dispersion liquid B-2 was 328 nm, and the CV value was 2.23%.

[Preparation Example 3]

<Synthesis of Platinum-Resin Composite Particles>

After adding 83.5 g of pure water to B-1 (30.00 g) obtained in Preparation Example 1, 7 wt% chloroplatinic acid aqueous solution (31.5 g) was added, and the mixture was stirred at 30°C at a stirring speed of 100 rpm to 200 rpm for 3 hours. This mixed solution was centrifuged, and excess chloroplatinic acid was removed by removing the supernatant. Thereafter, the concentration was adjusted to obtain a 5 wt% platinum ion-adsorbed resin particle dispersion liquid C-1.
Next, C-1 (70.45 g) diluted with 34.2 g of pure water was added to 4725 g of pure water while stirring at 0°C to 3°C at a stirring speed of 100 rpm to 200 rpm. Furthermore, while stirring at 0°C to 3°C at a stirring speed of 100 rpm to 200 rpm, 132 mM dimethylamine borane aqueous solution (138 g) was added dropwise over 20 minutes. Thereafter, by stirring at 0°C to 3°C at a stirring speed of 100 rpm to 200 rpm for 60 minutes, then at 25°C to 28°C at a stirring speed of 100 rpm to 200 rpm for 2 hours, platinum-resin composite particles D-1 having an average particle diameter of 438 nm were obtained. The CV value of the particle diameter in D-1 was 1.4%. After concentrating D-1, D-1 was purified by dialysis treatment, and the concentration was adjusted to obtain a 1 wt% platinum-resin composite particle dispersion liquid E-1. The absorbance of the platinum-resin composite particles F-1 in E-1 was 1.77. Moreover, the average particle diameter of the platinum particles in F-1 was 3 nm, the standard deviation of the particle diameter of the platinum particles was 0.9 nm, and the loading amount of platinum was 39.9 wt%. In these platinum-resin composite particles F-1, the platinum particles included encapsulated platinum particles completely encapsulated in the resin particles, and partially exposed platinum particles having portions embedded within the resin particles and portions exposed outside the resin particles, and at least some of the platinum particles were distributed three-dimensionally in the surface layer portion of the resin particles. As a result of particle size distribution measurement of the obtained E-1, the mode value $\alpha$ of the particle diameter was 438.1 nm, and the abundance ratio of particles smaller than the particle diameter of $\alpha \times 0.94$ was 1.0%.

[Preparation Example 4]

<Synthesis of Platinum-Resin Composite Particles>

A 5 wt% platinum ion-adsorbed resin particle dispersion liquid C-2 was obtained in the same manner as Preparation Example 3, except that B-2 obtained in Preparation Example 2 was used instead of B-1. Subsequently, platinum-resin composite particles D-2 having an average particle diameter of 368 nm were obtained in the same manner as Preparation Example 3. The CV value of the particle diameter in D-2 was 1.4%. After concentrating D-2, D-2 was purified by dialysis treatment, and the concentration was adjusted to obtain a 1 wt% platinum-resin composite particle dispersion liquid E-2. The absorbance of the platinum-resin composite particles F-2 in E-2 was 1.64. Moreover, the average particle diameter of the platinum particles in F-2 was 3 nm, the standard deviation of the particle diameter of the platinum particles was 0.9 nm, and the loading amount of platinum was 37.5 wt%. In these platinum-resin composite particles F-2, the platinum particles included encapsulated platinum particles completely encapsulated in the resin particles, and partially exposed platinum particles having portions embedded within the resin particles and portions exposed outside the resin particles, and at least some of the platinum particles were distributed three-dimensionally in the surface layer portion of the resin particles. As a result of particle size distribution measurement of the obtained E-2, the mode value $\alpha$ of the particle diameter was 368.0 nm, and the abundance ratio of particles smaller than the particle diameter of $\alpha \times 0.94$ was 2.0%.

[Preparation Example 5]

<Synthesis of Gold-Resin Composite Particles>

After adding 75.00 g of pure water to B-2 (30.00 g) obtained in Preparation Example 2, a 7 wt% chloroauric acid aqueous solution (42.9 g) was added, and the mixture was stirred at 30°C at a speed of 100 rpm to 200 rpm for 3 hours. This mixed solution was centrifuged, and excess chloroauric acid was removed by removing the supernatant. Thereafter, the concentration was adjusted to obtain a 5 wt% gold ion-adsorbed resin particle dispersion liquid C-3.
Next, C-3 (65.01 g) diluted with 30.0 g of pure water was added to 4725 g of pure water while stirring at 0°C to 3°C at a stirring speed of 100 rpm to 200 rpm. Furthermore, while stirring at 0°C to 3°C at a stirring speed of 100 rpm to 200 rpm, 528 mM dimethylamine borane aqueous solution (15.5 g) was added dropwise over 53 seconds. Thereafter, by stirring at 0°C to 3°C at a stirring speed of 100 rpm to 200 rpm for 60 minutes, then at 25°C to 28°C at a stirring speed of 100 rpm to 200 rpm for 2 hours, gold-resin composite particles D-3 having an average particle diameter of 342 nm were obtained. The CV value of the particle diameter in D-3 was 1.9%. After concentrating D-3, D-3 was purified by dialysis treatment, and the concentration was adjusted to obtain a 1 wt% gold-resin composite particle dispersion liquid E-3. The absorbance of the gold-resin composite particles F-3 in E-3 was 1.36. Moreover, the average particle diameter of the gold particles in F-3 was 19.2 nm, the standard deviation of the particle diameter of the gold particles was 31.1 nm, and the loading amount of gold was 48.3 wt%. In these gold-resin composite particles F-3, the gold particles included encapsulated gold particles completely encapsulated in the resin particles, and partially exposed gold particles having portions embedded within the resin particles and portions exposed outside the resin particles, and at least some of the gold particles were distributed three-dimensionally in the surface layer portion of the resin particles. As a result of particle size distribution measurement of the obtained E-3, the mode value $\alpha$ of the particle diameter was 342.0 nm, and the abundance ratio of particles smaller than the particle diameter of $\alpha \times 0.94$ was 2.0%.

[Preparation Example 6]

<Synthesis of Resin Particles>

A 500-milliliter round container was used, and an incomplete semicircular stirring blade was mounted so that the longitudinal direction was perpendicular to the central axis of the round container. Into this round container, 300 g of pure water, 80 wt% trioctylmethylammonium chloride aqueous solution (1.55 g, 3.07 mmol), and 50 wt% polyethylene glycol methyl ether methacrylate aqueous solution (10.00 g, 2.40 mmol) were added and dissolved. Next, 2-vinylpyridine (48.00 g, 457 mmol) and divinylbenzene (2.00 g, 15.4 mmol) were added, and the mixture was stirred under nitrogen flow at 30°C for 50 minutes, then at 60°C for 30 minutes at a stirring speed of 20 rpm. After stirring, 2,2-azobis(2-methylpropionamidine) dihydrochloride (0.250 g, 0.922 mmol) dissolved in 18.00 g of pure water was added dropwise, and the mixture was stirred at 60°C for 3 hours at a stirring speed of 20 rpm to obtain resin particles A-3. The particles were precipitated by centrifugation, the supernatant was removed, and then the particles were redispersed in pure water, followed by removal of impurities through filtration treatment. Thereafter, concentration adjustment was performed to obtain a 10 wt% resin particle dispersion liquid B-3. The average particle diameter of resin particles A-3 in resin particle dispersion liquid B-3 was 385 nm, and the CV value was 17.2%.

[Preparation Example 7]

<Synthesis of Platinum-Resin Composite Particles>

A 5 wt% platinum ion-adsorbed resin particle dispersion liquid C-4 was obtained in the same manner as Preparation Example 3, except that B-3 obtained in Preparation Example 6 was used instead of B-1. Subsequently, platinum-resin composite particles D-4 having an average particle diameter of 410 nm were obtained in the same manner as Preparation Example 3. The CV value of the particle diameter in D-4 was 18.6%. After concentrating D-4, D-4 was purified by dialysis treatment, and the concentration was adjusted to obtain a 1 wt% platinum-resin composite particle dispersion liquid E-4. The absorbance of the platinum-resin composite particles F-4 in E-4 was 1.75. Moreover, the average particle diameter of the platinum particles in F-4 was 3 nm, the standard deviation of the particle diameter of the platinum particles was 0.9 nm, and the loading amount of platinum was 38.5 wt%. In these platinum-resin composite F-4, the platinum particles included encapsulated platinum particles completely encapsulated in the resin particles, and partially exposed platinum particles having portions embedded within the resin particles and portions exposed outside the resin particles, and at least some of the platinum particles were distributed three-dimensionally in the surface layer portion of the resin particles. As a result of particle size distribution measurement of the obtained E-4, the mode value $\alpha$ of the particle diameter was 438.0 nm, and the abundance ratio of particles smaller than the particle diameter of $\alpha \times 0.94$ was 42.0%.

[Example 1]

(Antibody Binding Step)

After mixing 25 $\mu$g of anti-CRP antibody with 0.45 mL of 50 mM HEPES buffer (pH 7), 0.05 mL of 1 wt% platinum-resin composite particle dispersion liquid E-1 was added, and inversion stirring was performed at room temperature for 1 hour to obtain a labeled antibody dispersion liquid G-1 including anti-CRP antibody labeled with platinum-resin composite particles F-1.

(Blocking Step)

Next, the labeled antibody dispersion liquid G-1 was subjected to centrifugation at 3000 rpm for 5 minutes, and the supernatant was removed. Thereafter, 0.5 mL of HEPES buffer (pH 7) including 1 wt% sodium caseinate was added to the precipitated sediment, and after ultrasonic dispersion, inversion stirring was further performed at room temperature for 1 hour to obtain a labeled antibody dispersion liquid H-1.

(Washing Treatment)

Next, the labeled antibody dispersion liquid H-1 was subjected to centrifugation at 3000 rpm for 5 minutes, and the supernatant was removed. Thereafter, 0.5 mL of 5 mM Tris aqueous solution (pH 8.5) including less than 0.1 wt% surfactant was added to the precipitated sediment, and ultrasonic dispersion was performed. This operation was repeated 3 times to obtain a labeled antibody dispersion liquid I-1.

(Conjugate Pad Preparation)

The labeled antibody dispersion liquid I-1 was subjected to centrifugation at 3000 rpm for 5 minutes, and the supernatant was removed. Thereafter, 5 mM Tris aqueous solution (pH 8.5) including 5 wt% sucrose and 2.5 wt% BSA was added to the precipitated sediment, and ultrasonic dispersion was performed to obtain a labeled antibody dispersion liquid J-1. After uniformly impregnating glass fiber nonwoven fabric with labeled antibody dispersion liquid J-1, drying was performed at 50°C for 1 hour to prepare a conjugate pad K-1. At this time, the concentration of labeled antibody dispersion liquid J-1 and the coating amount on the glass fiber nonwoven fabric were adjusted so that the content of platinum-resin composite particles F-1 in conjugate pad K-1 became 3 $\mu$g per test in the evaluation by immunochromatography described later.

(Preparation of Immunochromatographic Strip)

An immunochromatographic strip having the structure shown in FIG. 4 was prepared. First, anti-CRP antibody was applied to a nitrocellulose membrane 4 having a width of 25 mm to draw a test line 5. Further, anti-mouse IgG antibody was applied to the downstream side of the test line 5 to draw a control line 6. After drying this nitrocellulose membrane 4 at 50°C for 1 hour, as shown in the cross-sectional view of the immunochromatographic strip in FIG. 4, a laminate film 1, a conjugate pad K-1 as the conjugate pad 3, a sample pad 2 (glass fiber nonwoven fabric), and an absorption pad 7 (cotton nonwoven fabric) were laminated. Finally, the strip was cut to a width of 3.5 mm to prepare an immunochromatographic strip L-1.

(Preparation of Development Solution)

An aqueous solution (pH 7.5) containing 50 mM Tris, 150 mM NaCl, 1.0 wt% BSA, and 1.0 wt% surfactant Triton X-100 was prepared and used as a development solution 1.

(Evaluation by Immunochromatography)

By diluting CRP antigen using the development solution 1, positive control (antigen concentration 78.0, 39.0, 19.5, 9.8, 4.9, 2.4, 1.2, 0.6, 0.3 ng/ml) and negative control (antigen-free) sample solutions were prepared. 50 $\mu$l of sample solution was dropped onto the sample pad 2 of immunochromatographic strip L-1. Thereafter, the color development intensity of the test line 5 after 10 minutes was measured with an immunochromatography reader (Hamamatsu Photonics C10066-10). The evaluation results by immunochromatography are shown in Table 1. The slope of test line color development intensity relative to antigen concentration in the low concentration range of antigen concentration 5 ng/mL or less was 5.7, and the correlation coefficient was 0.986.

[Example 2]

Antibody binding step, blocking step, washing treatment, conjugate pad preparation, immunochromatographic strip preparation, and immunochromatographic evaluation were performed in the same manner as in Example 1, except that 1 wt% platinum-resin composite particle dispersion liquid E-2 was used instead of 1 wt% platinum-resin composite particle dispersion liquid E-1. The evaluation results by immunochromatography are shown in Table 1. The slope of test line color development intensity relative to antigen concentration in the low concentration range of antigen concentration 5 ng/mL or less was 5.0, and the correlation coefficient was 0.989.

[Example 3]

Antibody binding step, blocking step, washing treatment, conjugate pad preparation, immunochromatographic strip preparation, and immunochromatographic evaluation were performed in the same manner as in Example 1, except that 1 wt% gold-resin composite particle dispersion liquid E-3 was used instead of 1 wt% platinum-resin composite particle dispersion liquid E-1. The evaluation results by immunochromatography are shown in Table 1. The slope of test line color development intensity relative to antigen concentration in the low concentration range of antigen concentration 5 ng/mL or less was 10.2, and the correlation coefficient was 0.989.

[Comparative Example 1]

Antibody binding step, blocking step, washing treatment, conjugate pad preparation, immunochromatographic strip preparation, and immunochromatographic evaluation were performed in the same manner as in Example 1, except that gold colloid (Tanaka Precious Metal, Au colloid solution-SC, particle diameter 50 nm) was used as particles, 5 mM Tris aqueous solution (pH 9.5) was used as binding buffer, and 5 mM Tris aqueous solution (pH 9.5) containing 1 wt% BSA was used as blocking buffer. Table 1 shows the evaluation results by immunochromatography. The slope of test line color development intensity relative to antigen concentration in the low concentration range of antigen concentration 5 ng/mL or less was 1.0, and the correlation coefficient was 0.779.

As a result of particle size distribution measurement of the gold colloid, the CV value of particle diameter was 8.2%, the mode value $\alpha$ of particle diameter was 48.7 nm, and the abundance ratio of particles smaller than the particle diameter of $\alpha \times 0.94$ was 17.0%.

[Comparative Example 2]

Antibody binding step, blocking step, washing treatment, conjugate pad preparation, immunochromatographic strip preparation, and immunochromatographic evaluation were performed in the same manner as in Example 1, except that 1 wt% platinum-resin composite particle dispersion liquid E-4 was used instead of 1 wt% platinum-resin composite particle dispersion liquid E-1. The evaluation results by immunochromatography are shown in Table 1. The slope of test line color development intensity relative to antigen concentration in the low concentration range of antigen concentration 5 ng/mL or less was 2.4, and the correlation coefficient was 0.884.

[Comparative Example 3]

Antibody binding step, blocking step, washing treatment, conjugate pad preparation, immunochromatographic strip preparation, and immunochromatographic evaluation were performed in the same manner as in Example 1, except that red latex (Merck Millipore, Estapor Red, K1 040D, particle diameter 400 nm) was used as particles and chemical bonding treatment by EDC-NHS coupling method was performed using 50 mM MES aqueous solution (pH 6) as binding buffer. Table 1 shows the immunochromatographic evaluation results. The slope of test line color development intensity relative to antigen concentration in the low concentration range of antigen concentration 5 ng/mL or less was 1.1, and the correlation coefficient was 0.779.

As a result of particle size distribution measurement of the red latex, the CV value of particle diameter was 20.3%, the mode value $\alpha$ of particle diameter was 338.8 nm, and the abundance ratio of particles smaller than the particle diameter of $\alpha \times 0.94$ was 18.0%.

[Table 1]

| CRP antigen concentration [ng/ml] | | 78.0 | 39.0 | 19.5 | 9.8 | 4.9 | 2.4 | 1.2 | 0.6 | 0.3 | 0.0 |
|---|---|---|---|---|---|---|---|---|---|---|---|
| Test line color development intensity [mABS] | Example 1 | 262.9 | 163.0 | 114.5 | 43.2 | 29.5 | 13.3 | 6.8 | 4.7 | 4.4 | 0.0 |
| | Example 2 | 230.7 | 150.3 | 100.2 | 41.5 | 25.5 | 11.0 | 6.5 | 4.7 | 2.1 | 0.0 |
| | Example 3 | 321.2 | 237.6 | 172.2 | 110.3 | 52.2 | 25.8 | 12.1 | 9.2 | 7.9 | 0.0 |
| | Comparative Example 1 | 134.5 | 74.4 | 44.3 | 19.0 | 11.6 | 0.0 | 0.0 | 0.0 | 0.0 | 0.0 |
| | Comparative Example 2 | 138.6 | 84.8 | 52.1 | 27.4 | 14.0 | 8.4 | 6.9 | 4.2 | 5.3 | 0.0 |
| | Comparative Example 3 | 60.2 | 39.0 | 21.7 | 9.2 | 5.7 | 0.0 | 0.0 | 0.0 | 0.0 | 0.0 |

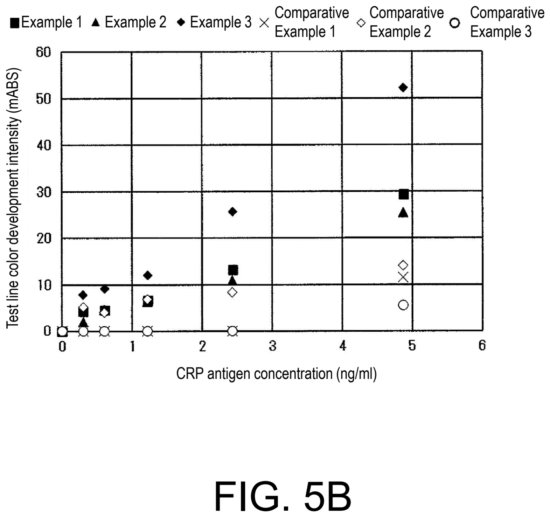

Based on the test results of Examples 1, 2, 3 and Comparative Examples 1, 2, 3 shown in Table 1, scatter plots of test line color development intensity relative to CRP antigen concentration (FIG. 5A, FIG. 5B) were created, and sensitivity comparison of immunochromatography was performed. Additionally, particle size distribution measurement results of particles used in Examples 1, 2, 3 and Comparative Examples 1, 2, 3, and the slope and correlation coefficient of test line color development intensity in the antigen concentration range of 0 ng/ml to 5 ng/ml were calculated and shown in Table 2.

As shown in FIG. 5A, in the case of using metal-resin composite particles with uniform particle diameter and CV value of 2% or less (Examples 1, 2, 3), test line color development intensity approximately twice that obtained in the case of using gold colloid or red latex (Comparative Examples 1, 3) was obtained, confirming that high-sensitivity detection can be achieved. Additionally, as shown in the scatter plot in the antigen concentration range of 0 ng/ml to 5 ng/ml in FIG. 5B and Table 2, in the low antigen concentration region, Examples 1, 2, 3 showed larger slopes in the calibration curve of test line color development intensity relative to antigen concentration and correlation coefficients close to 1, compared to Comparative Examples 1, 2, 3. From the above results, it can be said that in the case of using metal-resin composite particles with uniform particle diameter and CV value of 2% or less, it is possible to perform quantitative detection with higher sensitivity and lower antigen concentration.

[Table 2]

| | Particle size distribution measurement result | | | Calibration curve of antigen concentration in range of 0 ng/ml to 5 ng/ml | |
|---|---|---|---|---|---|
| | CV value | Mode value [nm] | Ratio of mode value×0.94 or less | Slope | Correlation coefficient |
| Example 1 | 1.4% | 438.1 | 1.0% | 5.7 | 0.986 |
| Example 2 | 1.4% | 368.0 | 2.0% | 5.0 | 0.989 |
| Example 3 | 1.9% | 342.0 | 2.0% | 10.2 | 0.989 |
| Comparative Example 1 | 8.2% | 48.7 | 17.0% | 1.0 | 0.779 |
| Comparative Example 2 | 18.6% | 438.0 | 42.0% | 2.4 | 0.884 |
| Comparative Example 3 | 20.3% | 338.8 | 18.0% | 1.1 | 0.779 |

Although the embodiments of the present invention have been described in detail above for illustrative purposes, the present invention is not limited to the above embodiments.

The present application claims priority based on Japanese Patent Application No. 2023-089651 filed in Japan on May 31, 2023, the entire contents of which are incorporated herein by reference.

Description of Reference Numerals

1...laminate film, 2...sample pad, 3...conjugate pad, 4...nitrocellulose membrane, 5...test line, 6...control line, 7...absorption pad, 10...resin particle, 20...metal particle, 30...encapsulated particle, 40...partially exposed particle, 50...surface adsorbed particle, 60...surface layer portion, 100...metal-resin composite, 110...membrane, 120...sample addition section, 130...determination section, 131...capture ligand, 140...liquid absorption section, 150...labeled antibody, 160...analyte, 170...composite, 200...test strip

**Claims**

1. A labeling substance, comprising a metal-resin composite that includes a resin particle and a plurality of metal particles immobilized on the resin particle,

   wherein a CV value of a particle diameter of the metal-resin composite is 6% or less, and
   in a case where a mode value of the particle diameter of the metal-resin composite detected by a particle size distribution analyzer is $\alpha$ [nm], an abundance ratio of particles having a particle diameter smaller than $\alpha \times 0.94$ is 10% or less.

2. The labeling substance according to claim 1, wherein the metal particles are particles of platinum or an alloy thereof, or particles of gold or an alloy thereof.

3. The labeling substance according to claim 1, wherein an average particle diameter of the metal-resin composite is within a range of 50 nm to 1000 nm.

4. The labeling substance according to claim 1, wherein at least some of the metal particles are distributed three-dimensionally in a surface layer portion of the resin particle.

5. The labeling substance according to claim 1, being used by adsorbing an antigen or antibody on a surface of the metal-resin composite.

6. An immunoassay method, using the labeling substance according to claim 1.

7. An immunoassay reagent, comprising the labeling substance according to claim 1.

8. A method for measuring an analyte, for detecting or quantifying the analyte contained in a sample, the method:

using a lateral-flow chromatographic test strip that comprises a membrane and a determination section in which a capture ligand that specifically binds to the analyte is immobilized on the membrane, and comprising following steps (I) to (III);
step (I): a step of bringing the analyte contained in the sample into contact with a labeled antibody in which an antibody that specifically binds to the analyte is labeled with the labeling substance according to claim 1,
step (II): a step of bringing a composite including the analyte and the labeled antibody, formed in step (I), into contact with the capture ligand in the determination section, and
step (III): a step of measuring a color development intensity derived from light energy absorption due to localized surface plasmon resonance and/or electron transition of the metal-resin composite in the labeling substance.

9. The method for measuring the analyte according to claim 8, further comprising step (IV): a step of estimating an amount of analyte contained in the sample based on the measured color development intensity.

10. An analyte measurement kit, for detecting or quantifying an analyte contained in a sample, the analyte measurement kit comprising:

a lateral-flow chromatographic test strip including a membrane and a determination section in which a capture ligand that specifically binds to the analyte is immobilized on the membrane; and
a detection reagent including a labeled antibody in which an antibody that specifically binds to the analyte is labeled with the labeling substance according to claim 1.

11. The analyte measurement kit according to claim 10, further comprising a device that measures a color development intensity in the determination section, wherein the color development intensity is derived from light energy absorption due to localized surface plasmon resonance and/or electron transition of the metal-resin composite in a composite of the analyte and the labeled antibody.

12. The analyte measurement kit according to claim 10, further comprising reference standard data showing a relationship between the color development intensity in the determination section and a concentration of the analyte, wherein the color development intensity is derived from light energy absorption due to localized surface plasmon resonance and/or electron transition of the metal-resin composite in a composite of the analyte and the labeled antibody.

13. A lateral-flow chromatographic test strip, for detecting or quantifying an analyte contained in a sample, the lateral-flow chromatographic test strip comprising:

a membrane;
a determination section in which a capture ligand that specifically binds to the analyte is immobilized on the membrane in a direction in which the sample develops; and
a reaction section including a labeled antibody in which an antibody that specifically binds to the analyte is labeled with the labeling substance according to claim 1 on an upstream side of the determination section.

FIG. 1

FIG. 2A

FIG. 2B

**FIG. 3**

**FIG. 4**

**FIG. 5A**

FIG. 5B

## INTERNATIONAL SEARCH REPORT

| International application No. |
| --- |
| **PCT/JP2024/019126** |

| A. | CLASSIFICATION OF SUBJECT MATTER |
| --- | --- |

*G01N 33/545*(2006.01)i; *G01N 33/543*(2006.01)i
FI:  G01N33/545 Z; G01N33/543 541Z; G01N33/543 521

According to International Patent Classification (IPC) or to both national classification and IPC

| B. | FIELDS SEARCHED |
| --- | --- |

Minimum documentation searched (classification system followed by classification symbols)

G01N33/545; G01N33/543

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2019-120497 A (NIPPON STEEL CHEMICAL & MATERIAL CO., LTD.) 22 July 2019 (2019-07-22)<br>paragraphs [0016]-[0018], [0022]-[0027], fig. 1-2 | 1-13 |
| A | WO 2017/010391 A1 (NIPPON STEEL & SUMIKIN CHEMICAL CO., LTD.) 19 January 2017 (2017-01-19)<br>paragraphs [0001], [0018]-[0021], [0025]-[0030], fig. 1-3 | 1-13 |
| A | JP 2005-098974 A (SEKISUI CHEMICAL CO., LTD.) 14 April 2005 (2005-04-14)<br>paragraphs [0008], [0022], [0023], [0033], [0039]-[0041], [0057], [0070], [0071], abstract | 1-13 |
| A | WO 2019/132045 A1 (SEKISUI CHEMICAL CO., LTD.) 04 July 2019 (2019-07-04)<br>paragraphs [0031], [0072]-[0088], tables 1-2 | 1-13 |
| A | JP 2017-040631 A (CHIBA UNIV.) 23 February 2017 (2017-02-23)<br>paragraphs [0001], [0023] | 1-13 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **27 June 2024** | **09 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/019126**

| C. | DOCUMENTS CONSIDERED TO BE RELEVANT | |
|---|---|---|
| Category* | Citation of document, with indication, where appropriate, of the relevant passages | Relevant to claim No. |
| A | WO 2019/059182 A1 (ASAHI KASEI KABUSHIKI KAISHA) 28 March 2019 (2019-03-28) paragraphs [0009]-[0010], [0019] | 1-13 |
| A | US 2014/0242720 A1 (FURUKAWA ELECTRIC CO., LTD.) 28 August 2014 (2014-08-28) paragraphs [0001], [0033], [0034], abstract | 1-13 |

Form PCT/ISA/210 (second sheet) (July 2022)

INTERNATIONAL SEARCH REPORT
Information on patent family members

International application No.

PCT/JP2024/019126

| Patent document cited in search report | | | Publication date (day/month/year) | Patent family member(s) | Publication date (day/month/year) |
|---|---|---|---|---|---|
| JP | 2019-120497 | A | 22 July 2019 | (Family: none) | |
| WO | 2017/010391 | A1 | 19 January 2017 | US 2018/0209965 A1 paragraphs [0001], [0025]-[0028], [0032]-[0040], fig. 1-3 KR 10-2020-0079559 A KR 10-2018-0030027 A EP 3869197 A1 EP 3321684 A1 CN 110542761 A CN 107850595 A | |
| JP | 2005-098974 | A | 14 April 2005 | (Family: none) | |
| WO | 2019/132045 | A1 | 04 July 2019 | US 2021/0061820 A1 paragraphs [0075]-[0080], [0142]-[0174], tables 1-2 EP 3733782 A1 | |
| JP | 2017-040631 | A | 23 February 2017 | (Family: none) | |
| WO | 2019/059182 | A1 | 28 March 2019 | US 2021/0080455 A1 paragraphs [0015]-[0017], [0019] KR 10-2020-0028445 A CN 111108387 A | |
| US | 2014/0242720 | A1 | 28 August 2014 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009168495 A
- WO 2016002742 A
- WO 2017010391 A
- JP 2023506679 A

- JP 2022502637 A
- JP 6833519 B
- JP 2023089651 A

**Non-patent literature cited in the description**

- **K. AKAMATSU ; M. SHIMADA ; T. TSURUOKA ; H. NAWAFUNE ; S. FUJII ; Y. NAKAMURA**. *Langmuir*, 2010, vol. 26, 1254-1259